# EUROPEAN PATENT SPECIFICATION

(11) **EP 3 267 880 B1**
(45) Date of publication and mention of the grant of the patent: **14.11.2018**
(21) Application number: 16712495.7
(22) Date of filing: 12.02.2016
(51) Int. Cl.: A61B 5/024

(54) **PORTABLE ELECTRONIC DEVICE TO PROCESS A SIGNAL ACQUIRED FROM A LIVING BODY AND METHOD THEREOF**
TRAGBARE ELEKTRONISCHE VORRICHTUNG ZUR VERARBEITUNG EINES VON EINEM LEBENDEN KÖRPER ERFASSTEN SIGNALS UND VERFAHREN DAFÜR
DISPOSITIF ÉLECTRONIQUE PORTABLE POUR TRAITER UN SIGNAL ACQUIS À PARTIR D'UN CORPS VIVANT, ET PROCÉDÉ ASSOCIÉ

(30) Priority: 12.03.2015 WO PCT/EP2015/000550
(43) Date of publication of application: 17.01.2018
(73) Proprietor: MICROBIOMED S.R.L., 88100 Catanzaro (IT)
(72) Inventor: VESCIO, Basilio, 88040 Platania (CZ) (IT); QUATTRONE, Aldo, 89100 Reggio Calabria (IT); GAMBARDELLA, Antonio, 88046 Lamezia Terme (CZ) (IT); SALSONE, Maria, 87027 Paola (CS) (IT)
(74) Representative: Dondi, Silvia
(86) International application number: PCT/IB2016/050758
(87) International publication number: WO 2016/142793

(56) References cited:
- WO-A2-2011/130541
- WO-A2-2011/150362
- US-A1- 2009 076 399

## Description

### TECHNICAL FIELD OF THE INVENTION

The present invention generally relates to the field of processing a signal acquired from a living body.

More in particular, the present invention relates to a portable electronic device for the automatic detection of behaviour disorders during the REM (Rapid-Eye Movement) sleep phase and, preferably, of sleep apnea.

### PRIOR ART

There is a known sleep disorder characterized by phenomena of motor agitation (for example, shouting and kicking) during the REM sleep phase and commonly referred to as RBD (abbreviation of "REM Sleep Behaviour Disorder").

In a subject who reports sleep disturbances that are not well defined, it is important to assess the presence of behavioural disorders in the REM sleep phase, since ascertaining the presence of RBD will make it possible to implement a therapeutic strategy aimed at overcoming the disorder itself and evaluating the risk of later developing Parkinson's disease, which can also be diagnosed after 15 years (after 7-8 years on average).

It is known that over 3% of the population suffers from obstructive sleep apnea syndrome (abbreviated OSAS).

OSAS is a fairly widespread disorder in which breathing is interrupted one or more times, or slows excessively during non-REM sleep; the pauses can last from at least 10 seconds to less than 3 minutes.

OSAS is said to occur when the number of apnea episodes is equal to or greater than 5 in one hour, or when there is a number of events equal to or greater than 15 per night.

A subject who suffers from apnea usually snores in a highly evident manner from the earliest stages of sleep, but not all snorers have OSAS. After the apnea episode, breathing usually resumes with sonorous snoring.

OSAS can provoke daytime drowsiness, with a reduction in attention capacity and possible impairment of driving capacity. For example, Italian law (Legislative Decree of 22 December 2015, Official Gazette of 13 January 2016) provides that a driving license cannot be issued to candidates or renewed for drivers affected by OSAS.

In a subject who reports sleep disturbances that are not well defined, it is important to assess whether sleep apnea is present, since ascertaining the presence of OSAS will make it possible to implement a therapeutic strategy aimed at overcoming the disorder itself and evaluating the capacity to drive motorvehicles.

There is a known medical device referred to as a polysomnograph which allows several physiological parameters of a subject to be recorded simultaneously during sleep, typically two or more electroencephalogram channels, various electromyographic channels, chest and abdominal movements, oronasal flow and blood oxygen saturation.

Physiological parameters are normally measured by means of electrodes or other systems that are fixed to the patient's body and connected with cables to a base system which receives, amplifies and analyzes the signals (electrodes for electroencephalograms, electrodes for electromyography, chest strap and sensor for breathing, electrodes for electrocardiograms, etc).

The polysomnograph allows to detect the presence of RBD and OSAS, but it has a disadvantage in that it must be used in a hospital environment, since it is a cumbersome and costly device and further requires the presence of specialized medical and technical personnel for the application of the detectors and for reading and interpretation of the results.

Due to the encumbrance of electrodes and cables attached to the patient and connected to the basic processing system, it has the further disadvantage of making it difficult for the subject to fall asleep and reach and maintain an optimal quality and quantity of sleep (with the presence of REM phases), with a consequent possible invalidity of the test, which will thus have to be repeated a number of times, causing discomfort for the subject and an increase in cost.

There are some known portable medical devices, such as, for example, the "SOMNOscreen plus PSG" by SOMNOmedics GmbH (www.somnomedics.de): said devices have reduced dimensions and can thus be used at the patient's home, but they still require the presence of specialized medical and technical personnel for the application of the detectors and interpretation of the results.

The portable medical devices are also conceived like the traditional polysomnograph and pose the same problems in terms of connections between the parameters sensors and the central unit, with the difficulty of reaching and maintaining optimal sleep.

There are known portable medical devices (cardiorespiratory polygraphy) designed for the study of sleep apnea, such as, for example "SOMNOtouch RESP" by SOMNOmedics GmbH; these devices allows to measure the amount of oxygen in the blood, the movement of air in the nose during breathing, heart rate, chest movements during breathing, the position of the body and other parameters, and they can be used at the patient's home, but require the presence of technical or medical personnel for their application and for the interpretation of the results and pose the problems previously described of being cumbersome and making it difficult to reach and maintain optimal sleep.

Furthermore, the portable medical devices do not enable automatic detection of the parameters indicative of RBD.

From WO 2011/150362 it is known a sleep apnea detection system. When a user sleeps, sounds can be picked up by a microphone and recorded. During or after this recording, the sounds are analyzed to determine whether the individual suffers from sleep apnea.

### SUMMARY OF THE INVENTION

The present invention relates to an electronic device for the automatic detection of behaviour disorders during the REM sleep phase and sleep apnea as defined in the enclosed claim 1 and by its preferred embodiments disclosed in the dependent claims from 2 to 12.

The Applicant has perceived that the electronic device according to the present invention has the following advantages:
- it allows automatic detection of biological parameters indicative of sleep behaviour disorders (for example, of the RBD type) and, preferably, sleep apnea, for large-scale screening applications;
- it can be used at the home of a subject who reports sleep disorders that are not well defined; it does not require the presence of specialized medical or technical personnel;
- it is compact;
- it is flexible;
- it can be easily worn and used by the subject himself or herself and does not need to be applied by technical or medical personnel;
- it provides a result that can be easily interpreted by the subject without the aid of medical personnel;
- it is not cumbersome and does not cause difficulties in falling asleep or maintaining a qualitatively and quantitatively optimal sleep, and thus enables a valid test;
- it is inexpensive.

It is also an object of the present invention an electronic system as defined in the enclosed claim 13 and by its preferred embodiments disclosed in the dependent claims 14 and 15.

It is also an object of the present invention a method for processing a signal representative of a variation in volume of the blood flow of a human tissue of the living body, as defined in the enclosed claim 16 and by its preferred embodiments disclosed in the dependent claims 17 to 23.

It is also an object of the present invention a computer program as defined in the enclosed claim 24.

### BRIEF DESCRIPTION OF THE DRAWINGS

Figure 1 shows a block diagram of an electronic system for the automatic detection of behaviour disorders during the REM sleep phase and sleep apnea according to the invention.
Figure 2A shows more in detail the block diagram of an electronic device used in the electronic system of Figure 3.
Figure 2B shows more in detail the block diagram of a processing module inside a processing unit of the electronic device of the invention.
Figure 3 shows an example of the external shape of the casing of the electronic device according to the invention.
Figures 4A-4B show the flow chart of a method for processing a signal acquired from a living body according to a first embodiment of the invention.
Figures 5A-5B show the flow chart of a method for processing a signal acquired from a living body according to a second embodiment of the invention.

### DETAILED DESCRIPTION OF THE INVENTION

Note that in the description below, identical or similar blocks, components or modules are indicated by the same numerical references in the figures.

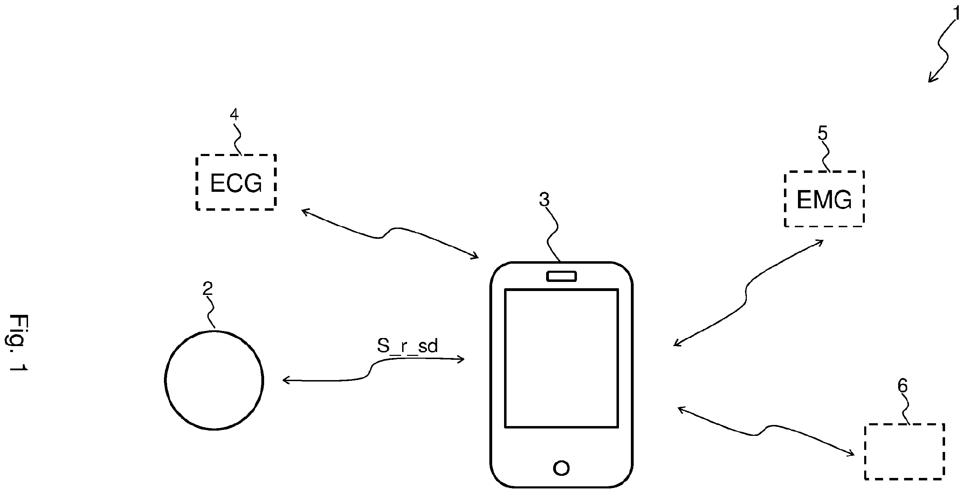

With reference to figure 1, it shows an electronic system 1 for processing a signal acquired from a living body according to the invention; in particular, the signal is acquired when a subject is sleeping and/or awake, during the day and/or during the night.

The electronic system 1 comprises a portable electronic device 2 and a mobile electronic device 3, which are connected to each other by means of a short-range wireless signal S_r_sd (for example, a radio signal of the Bluetooth, WiFi or ZigBee type).

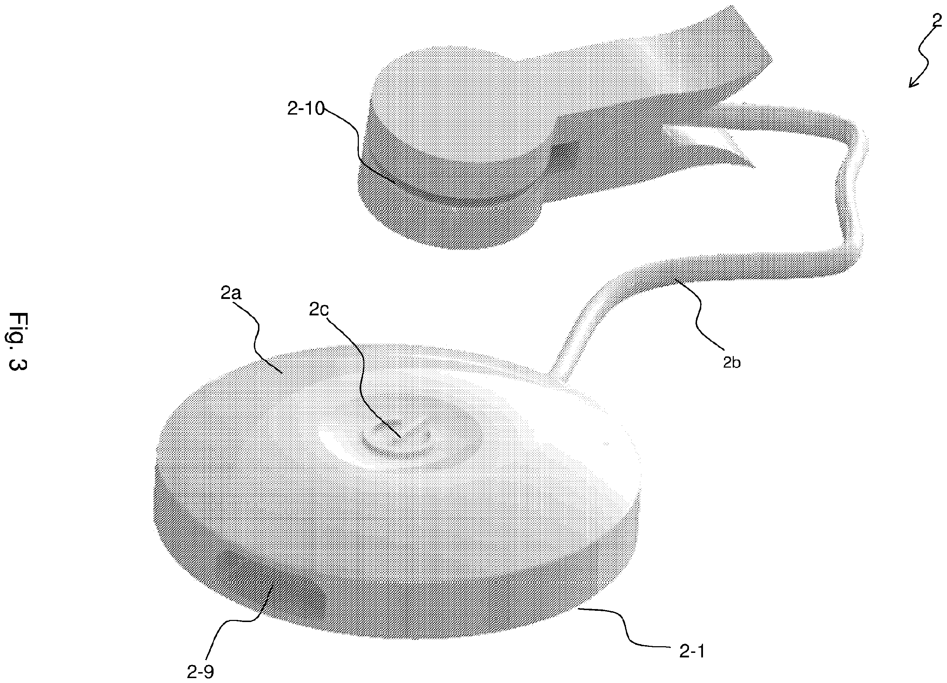

The portable electronic device 2 has compact dimensions which allows an easy transport and use (both during daytime activities and during the night) by a subject who complains of sleep disturbances that are not well defined, as will be illustrated in greater detail below in relation to the description of Figure 3.

The mobile electronic device 3 comprises a screen to display the signals generated by the portable electronic device 2; preferably, the mobile electronic device 3 is configured to process the data acquired from the portable electronic device 2.

The mobile electronic device 3 is selected, for example, from the following:
- a smartphone (for example, an iPhone);
- a tablet (for example, an iPad);
- a desktop or laptop computer.

Preferably, the electronic system 1 further comprises one or more additional body detectors 4, 5, 6 connected to the mobile electronic device 3 by means of short-range wireless signals (for example, of the Bluetooth, WiFi or ZigBee type), as will be explained in greater detail below.

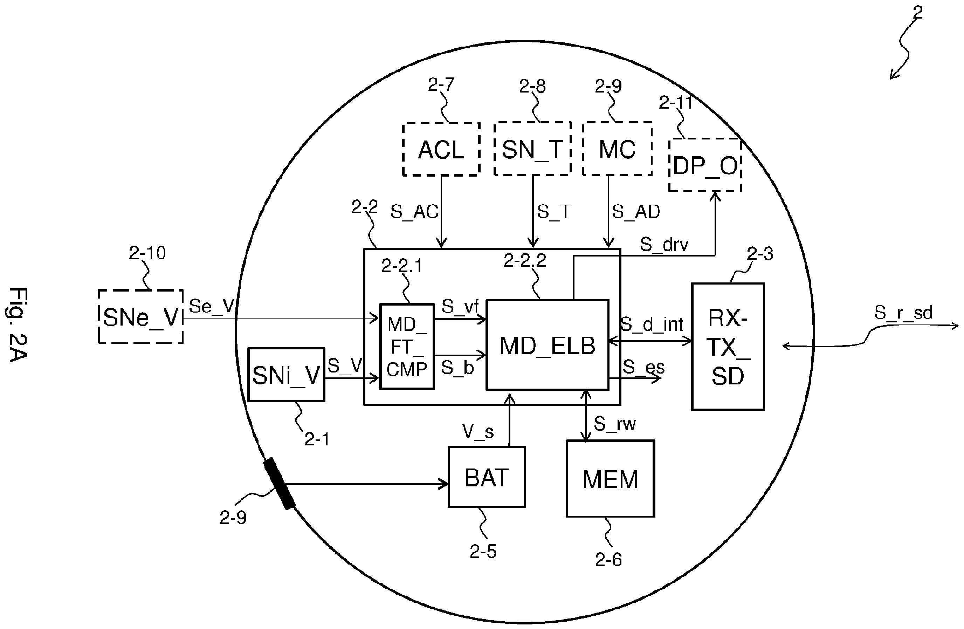

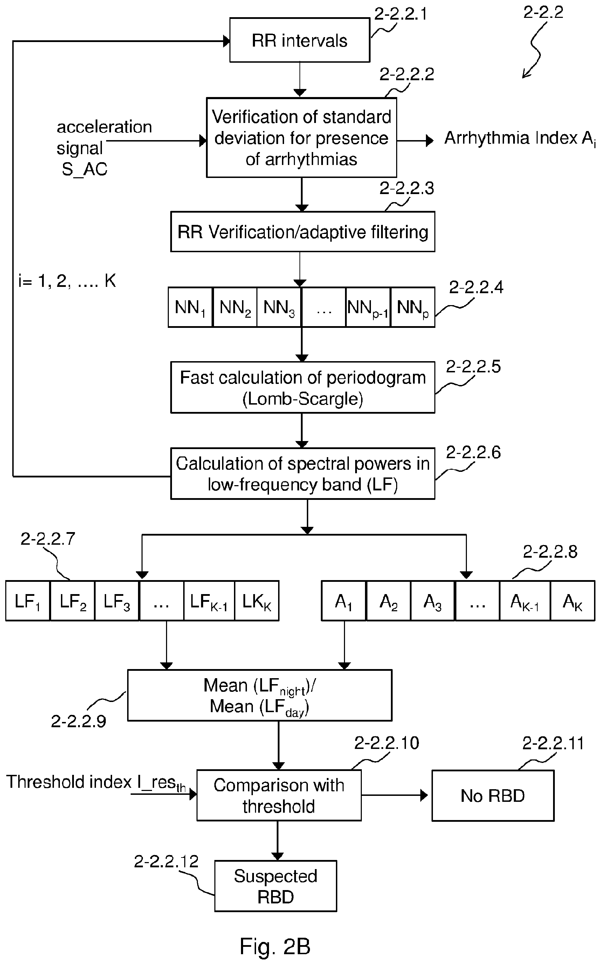

With reference to Figure 2, it shows more in detail the block diagram of the portable electronic device 2.

The portable electronic device 2 comprises:
- an internal sensor 2-1;
- a processing unit 2-2;
- a transceiver 2-3 of short-range wireless signals;
- a battery 2-5;
- a memory 2-6.

Preferably, the portable electronic device 2 further comprises an output device 2-11 having the function of generating a visual, audio or text indication indicative of the result of the processing carried out by means of the processing unit 2-2.

In particular, the output device 2-11 is a light-emitting diode (LED). In this case the processing unit 2-2 is configured to further generate a driving signal S_drv for the light-emitting diode 2-11, which can take on the following colours:
- steady green light: it indicates the absence of sleep behaviour disorder (in particular, of the RBD type) and the absence of sleep apnea;
- steady yellow light: it indicates the absence of sleep behaviour disorder (RBD) and a suspected presence of sleep apnea;
- steady blue light: it indicates a suspected presence of sleep behaviour disorder (RBD) and a suspected presence of sleep apnea;
- steady red light: it indicates a suspected presence of sleep behaviour disorder (RBD) and an absence of sleep apnea.

The presence of the light-emitting diode allows to interpret the result of the test in a simple manner by means of a light signal.

Alternatively, the output device 2-11 is a audio diffuser having the function of generating a sound signal indicative of the result of the processing carried out by means of the processing unit 2-2.

Preferably, the electronic device 2 further comprises a temperature sensor 2-8 and a miniaturized microphone 2-9.

The portable electronic device 2 has the function of automatically detecting biological parameters indicative of a sleep behaviour disorder (RBD) of a subject.

In particular, the portable electronic device 2 allows to automatically detect biological parameters indicative of sleep disorders of the RBD type and to perform a screening of subjects who may be candidates for a more in-depth test such as polysomnography.

Preferably, the portable electronic device 2 further allows to automatically detect biological parameters indicative of sleep apnea in the subject being analyzed, confirmable if necessary by cardiorespiratory polygraphy.

The internal sensor 2-1 has the function of generating an acquisition signal S_V representative of the variation in the volume of blood flow of a human tissue of the living body.

The internal sensor 2-1 is positioned inside the main body 2a of the portable electronic device 2 (see Figure 3), in particular above an inside surface of the base of the main body 2a.

The sensor 2-1 is applied in contact with or in proximity to a tissue of the human body of the subject being analyzed, such as, for example, on an earlobe or on a finger.

Preferably, the portable electronic device 2 further comprises an external sensor 2-10 and an interface for connecting the external sensor 2-10 to the portable electronic device 2 by means of an electrical connection cable 2b detachable from the main body 2a.

The external sensor 2-10 has a function similar to that of the internal sensor 2-1; in particular, the external sensor 2-10 is configured to generate a further acquisition signal Se_V representative of the variation in the volume of the blood flow of a human tissue of the living body.

When the external sensor 2-10 is connected, the operation of the internal sensor 2-1 is disabled.

The external sensor 2-10 is positioned, for example, inside a clip-on unit (see Figure 3), which can be for example secured to the earlobe or auricle.

The transceiver 2-3 comprises an input/output terminal adapted to receive the short-range wireless signal S_r_sd from the mobile electronic device 3, process it and forward it to the processing unit 2-2 on the internal data signal S_d_int.

Moreover, the transceiver 2-3 is configured to receive the internal data signal S_d_int from the processing unit 2-2, process it and forward it on the short-range wireless signal S_r_sd.

The battery 2-5 has the function of generating a voltage signal V_s for powering the electronic components of the portable electronic device 2, i.e. the processing unit 2-2, the sensors 2-1 and 2-10, the memory 2-6, the light-emitting diode, the temperature sensor 2-8 and the miniaturized microphone 2-9, for periods even longer than 24 hours.

The memory 2-6 has the function of storing a plurality K of power spectral values P₁, P₂, ... P_{K} calculated during a total measuring time interval T_mt, which can be comprised between a few hours and a few days, as will be explained in greater detail below.

Moreover, the memory 2-6 is configured to store:
- a plurality *p* of samples C₁, C₂ ... Cₚ of interbeat intervals;
- a validity flag associated with each value of the interbeat intervals.

The processing unit 2-2 is such to run a software application to perform a processing of a signal acquired from a living body, in particular to automatically detect biological parameters indicative of sleep behaviour disorders of a subject (for example of the RBD type) and, preferably, biological parameters indicative of sleep apnea: this allows to perform a screening of subjects who may be candidates for a more in-depth test, as will be explained in greater detail below.

The processing unit 2-2 is for example a microcontroller or a microprocessor.

The processing unit 2-2 comprises:
- a first input terminal adapted to receive the acquisition signal S_V from the sensor 2-1;
- a second input terminal adapted to receive the voltage signal V_s for powering the processing unit 2-2 from the battery 2-5;
- at least one input/output terminal for reading/writing the data from/in the memory 2-6;
- an input/output terminal adapted to transmit to the transceiver 2-3 an internal data signal S_d_int carrying the data processed by the processing unit 2-2 and adapted to receive from the transceiver 2-3 the internal data signal carrying data indicative of commands to be executed by the processing unit.

Preferably, the processing unit 2-2 further comprises an output terminal adapted to generate a processing result signal S_es representative of the result of the processing of the signal acquired from the living body; in particular, the processing result signal S_es is representative of a suspected presence or of an absence of a sleep behaviour disorder of the subject being analyzed (for example of the RBD type) and, preferably, is indicative of a presence or absence of a suspected presence of sleep apnea.

In this case the processing result signal S_es is the driving signal S_drv adapted to drive the light-emitting diode 2-11, which is configured to generate, as a function of the values of the driving signal S_drv, green or yellow or blue or red light, either of a steady type or intermittent with variable frequency.

The processing unit 2-2 is configured to calculate, in a defined measurement time interval and as a function of the values of the acquisition signal S_V, a plurality *p* of samples C₁, C₂,... Cₚ representative of a respective plurality *p* of interbeat intervals between adjacent heartbeats of the subject being analyzed.

The term "interbeat interval" means the time interval comprised between two adjacent heartbeats.

Advantageously, the processing unit 2-2 is further configured to calculate the oxygen saturation percentage in the blood of the subject being analyzed, as a function of the values of the acquisition signal S_V, in order to verify whether there is a suspected sleep apnea in the subject being analyzed.

In this case the memory 6 is further configured to store the calculated percentage values of oxygen saturation during the considered total measurement time interval T_mt (for example, hours or days).

The processing unit 2-2 in turn comprises a series connection of a filter and comparison module 2-2.1 and a processing module 2-2.2.

The filter and comparison module 2-2.1 is configured to receive the measurement signal S_V from the sensor 2-1 and to generate, as a function thereof, a filtered measurement signal S_vf and a beat sequence signal S_b carrying a pulse sequence representative of the heartbeats of the subject being analyzed.

More in particular, the filter and comparison module 2-2.1 comprises a filter sub-module and a comparison sub-module.

The filter sub-module is configured to receive the acquisition signal S_V as input and to perform a band-pass filtering with a bandwidth of between 1 Hz and 3 Hz, thus generating the filtered measurement signal S_vf as output.

Preferably, said band-pass filtering is achieved with a cascade connection of two filters, a high-pass one and a low-pass one.

The comparison sub-module is configured to receive the acquisition signal S_V as input and to generate, as a function thereof, the beat sequence signal S_b carrying a pulse sequence representative of the heartbeats of the subject being analyzed.

Advantageously, the processing unit and the transceiver 2-3 are implemented with a single programmable integrated circuit of the "System-on-chip" type, such as, for example, component CY8C4247LQI by Cypress.

The processing module 2-2.2 is configured to receive the beat sequence signal S_b and to calculate, as a function thereof, a plurality *p* of samples C₁, C₂,... Cₚ representative of a respective plurality *p* of interbeat intervals T₁, T₂, ... Tₚ between adjacent heartbeats of the subject being analyzed.

Moreover, the processing module 2-2.2 is configured to receive the filtered measurement signal S_vf and the beat sequence signal S_b and is configured to generate, as a function thereof and of the value of the data read by the memory 2-6, the internal data signal S_d_int and, preferably, the processing result signal S_es.

Advantageously, the sensor 2-1 is a photoplethysmographic sensor, which has the advantage of being particularly compact; in this case the acquisition signal S_V is a plethysmographic signal indicative of electric potentials generated on the human tissue (on which it is applied) by cardiac cellular activity.

In particular, the photoplethysmographic sensor 2-1 comprises a first light-emitting diode (LED) in the red band, a second light-emitting diode in the infrared band and a photodetector (for example, a photodiode).

In this case, the comparison sub-module is configured to receive the plethysmographic signal S_V as input and to perform a comparison with a threshold value so as to generate the beat sequence signal S_b having a square wave pattern representative of the heartbeats of the subject being analyzed.

Preferably, the photoplethysmographic sensor 2.1 has a planar shape and is of the reflectance type, i.e. it is such as to detect (by means of the photodetector) the variation in the red and infrared light reflected by the skin tissue on which the photoplethysmographic sensor 2-1 rests.

In this case the processing unit 2-2 comprises a further input terminal adapted to receive from the external sensor 2-10 the further acquisition signal Se_V.

Preferably, the sensor 2-10 is also a photoplethysmographic sensor and can be of the reflectance or penetrating type:
- in the case of a photoplethysmographic sensor 2-10 of the reflectance type, it is of a planar type and it is such to detect (by means of the photodetector) the variation of red light and infrared reflected from the skin tissue on which the photoplethysmographic sensor 2-10 rests;
- in the case of a photoplethysmographic sensor 2-10 of the penetrating type, it is of a vertical type and is such to detect (by means of the photodetector) the variation in the part of red and infrared light that passes through the skin tissue on which the photoplethysmographic sensor 2-10 rests.

Preferably, the photoplethysmographic sensors 2-1 and 2-10 are structured in such a way that the connection of the external sensor 2-10 (of the reflectance or penetrating type) is such to disable the internal sensor 2-1 of the reflectance type.

It should be noted that the beat sequence signal S_b representative of the heartbeats of the subject being analyzed can also be obtained using sensors 2-1 and 2-10 different than a photoplethysmographic sensor, provided that said sensors are capable of detecting heart activity and of correlating it with an electric signal S_V.

For example, sensors of an optical, pneumatic or radiofrequency type can be used.

In particular, the sensor 2-1 (and/or 2-10) is a set of one or more electrodes (for example, three electrodes) such to generate respective electrical signals representative of the current generated on the skin by the heart activity of a human being in the respective points in which said one or more electrodes are positioned; in this case the beat sequence signal S_b generated by the comparison sub-module is a electrocardiographic signal and the processing sub-module 2-2.2 is such to calculate the interbeat interval by measuring the distance between two adjacent R waves of the electrocardiographic signal.

Advantageously, the portable electronic device 2 further comprises an accelerometer 2-7 with at least one axis and configured to generate an acceleration signal S_AC representative of the acceleration value of the portable electronic device 2 along the at least one axis.

In this case the memory 2-6 is further configured to store the values of the acceleration signal S_AC during the considered total measurement time interval T_mt (for example, hours or days).

The use of the accelerometer 2-7 allows to compensate for the presence of any artifacts present in the acquired signal S_V by means of an adaptive filtering, as will be explained in greater detail below.

Preferably, the accelerometer 2-7 has three axes and it is thus such to generate the acceleration signal S_AC representative of three acceleration values of the portable electronic device 2 along the three axes.

Advantageously, the use of the accelerometer 2-7 further allows to identify states of agitation of the subject during sleep, which is useful for supporting the suspicion of RBD.

In this case the processing unit 2-2 is configured to receive and analyze the acceleration signal S_AC of the accelerometer 2-7 for the purpose of evaluating the state of movement during sleep and the correlation between state of agitation and emission of vocal sounds (shouts, moans, muttering, etc.) and/or snoring.

Advantageously, the processing unit 2-2 is configured to compare the energy of the signal coming from the accelerometer with a preset threshold and to detect the exceeding of that threshold for periods longer than 60 seconds in order to identify episodes of agitation during sleep.

The episodes of agitation, especially if accompanied by the emission of vocal sounds (shouts, moans, muttering etc.), of a duration comprised between 1 and 20 minutes, are detected and stored by means of the accelerometer 2-7 and the microphone 2-9 and are strongly indicative of RBD.

Advantageously, the portable electronic device 2 further comprises a temperature sensor 2-8 configured to generate a temperature signal S_T representative of a temperature of the living body of the subject being analyzed.

In this case the memory 2-6 is further configured to store the values of the temperature signal S_T during the considered total measurement time interval T_mt (in particular, 24 hours).

The presence of the temperature sensor 2-8 allows to verify whether the subject being analyzed is sleeping or not, based on significant variations in the temperature values.

Preferably, the miniaturized microphone 2-9 is configured to detect snoring and/or episodes of emission of vocal sounds (shouts, moans, muttering, etc.) by the subject being analyzed and to generate an audio signal S_AD of an electrical type representative of said snoring or emission of said vocal sounds

In this case the processing unit 2-2 is further configured to detect, as a function of the values of the audio signal S_AD generated by the microphone 2-9, the presence of snoring during sleep or at the end of the apnea, in order to confirm the state of sleep or the presence of a suspected apnea in the subject being analyzed.

Analogously, the processing unit 2-2 is further configured to detect, based on the values of the audio signal S_AD generated by the microphone 2-9, the presence of episodes of emission of vocal sounds (such as shouts, moans, muttering, etc.) during sleep, in order to support the suspicion of RBD.

Preferably, the processing unit 2-2 is capable of distinguishing between snoring and vocal sounds (shouts, moans, muttering, etc.) by means of a spectral analysis on different frequency bands, or alternatively by means of artificial intelligence techniques (such as, for example, neural networks, support vector machines (SVMs) or Bayesian models).

Advantageously, the processing unit 2-2 and/or the device 3 can timing correlate the various information detected by the sensors 2-1 (or 2-10), 2-7 and 2-9, which is useful for the purpose of increasing accuracy in the identification of RBD or sleep apnea, such as for example:
- simultaneous detection of the termination of snoring and decrease in O2 saturation (defined as phenomenon 1), indicative of the suspected presence of sleep apnea; or
- simultaneous detection of a state of agitation and emission of vocal sounds (such as shouts, moans, muttering, etc.) for a duration of between 1 and 20 minutes (defined as phenomenon 2), to support the suspected presence of RBD.

With reference to Figure 3, it shows the external shape of the casing of the portable electronic device 2, comprising:
- a main body 2a having a substantially circular disk-shaped casing having a diameter of about 25 millimetres;
- the reflectance photoplethysmographic sensor 2-1 applied at the base of the main body 2a;
- the photoplethysmographic sensor 2-10 (of the reflectance or penetrating type) inserted in a clip-on unit that can be for example secured to an earlobe;
- a cable 2b, disengageable from the body 2a, for the electrical connection between the external sensor 2-10 and the processing unit inside the main body 2a and such to carry the plethysmographic signal S_V;
- a button 2c for turning on and off the portable electronic device 2.

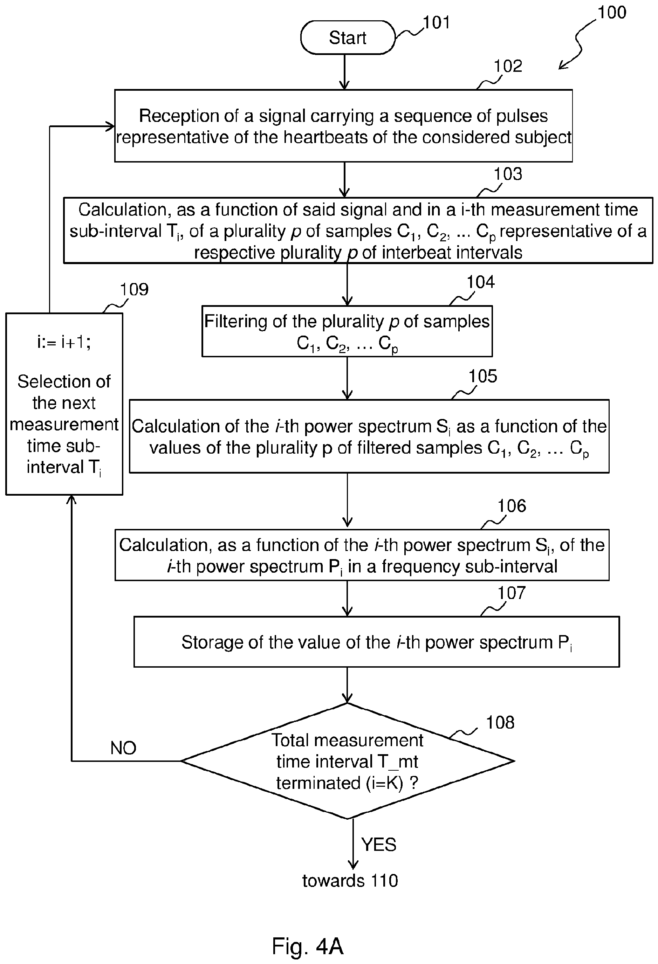

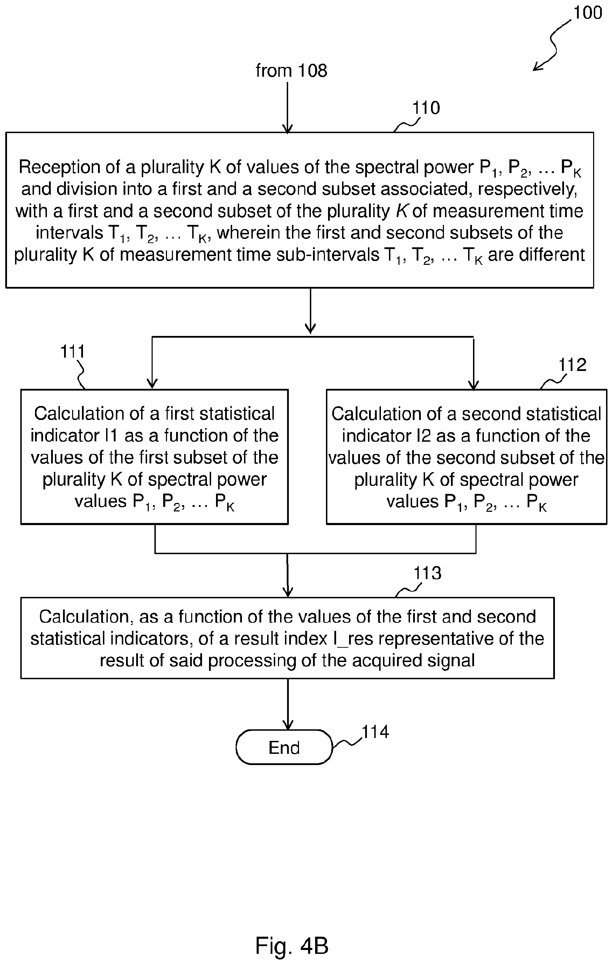

With reference to Figures 4A-4B, they show a flow chart 100 of the method for processing the signal S_V acquired from a living body of a subject according to a first embodiment of the invention.

The flow chart 100 is implemented by means of a software program running on the processing unit 2-2, in particular in the processing module 2-2.2.

The flow chart 100 starts with step 101.

From step 101 the method proceeds to step 102, wherein a signal is received carrying a pulse sequence representative of the heartbeats of the subject being analyzed.

As previously explained, said signal is generated by means of the sensor 2-1 and of the processing unit 2-2, in particular by the comparison sub-module that generates the beat sequence signal S_b carrying a series pulses representative of the heartbeats of the subject being analyzed.

From step 102 the method proceeds to step 103, wherein it is calculated, as a function of the values of said signal and in an i-th (i=1, 2, ... K) measurement time sub-interval Tᵢ, a plurality *p* of samples C₁, C₂, ... Cₚ representative of a respective plurality *p* of interbeat intervals (for example, *p*=300).

As previously explained, said plurality *p* of samples C₁, C₂, ... Cₚ is calculated by the processing module 2-2.2.

From step 103 the method proceeds to step 104, wherein it is perfomed the filtering of the plurality *p* of samples C₁, C₂, ... Cₚ, in order to verify whether the values of the interbeat interval have precise values and variations over time according to a pattern compatible with that of the heart of a living individual: this allows to identify mechanical artifacts (due to impacts or movements) or heartbeat irregularities (for example, permanent arrhythmias).

In particular, it is verified whether the precise values of the plurality *p* of samples C₁, C₂, ... Cₚ are within a range of values comprised between a minimum interbeat value (for example, 0.4 seconds) and a maximum interbeat value (for example, 1.8 seconds); alternatively (or in combination), it is verified whether the variation between two adjacent values of the plurality *p* of samples C₁, C₂, ... Cₚ is less than a maximum value of interbeat variation (for example, equal to 0.4 seconds).

Moreover, the validity flag (associated with each value of the interbeat intervals) is assigned to a first value (for example, a high logical value) to indicate that the respective sample Cᵢ is valid, whereas it is assigned to a second value (in the example, a low logical value) to indicate that the respective sample Cᵢ is not valid.

Advantageously, in step 104 it is received the acceleration signal S_AC (generated by the accelerometer 2-7) representative of the acceleration value of the portable electronic device 2 along the at least one axis.

In this case step 104 comprises the following additional sub-steps:
- calculating, based on the acceleration value signal S_AC, an energy index representative of the energy on at least one axis in at least one time interval comprised between two adjacent heartbeats;
- detecting, based on a comparison between the value of the calculated energy index and a idle energy threshold, the presence or absence of a state of agitation of the subject considered during sleep.

The value of the idle energy threshold is calculated, for example, during the stage of initialization of the portable electronic device 2.

Preferably, in step 104 a filtering of an adaptive type is also carried out on at least a part of the values of the plurality *p* of calculated samples C₁, C₂, ... Cₚ by means of the processing the acceleration signal generated by the accelerometer 2-7, as illustrated, for example, in the document by Ming-Zher Poh et al., "Motion-Tolerant Magnetic Earring Sensor and Wireless Earpiece for Wearable Photoplethysmography", IEEE Transactions on Information Technology in Biomedicine, Vol.14, no.3, May 2010, pages 786-794: this has the advantage of automatically compensating, in real time, for the artifacts caused by movement, thus providing correct interbeat intervals representative of the heart's activity during the normal everyday activities.

From step 104 the method proceeds to step 105, wherein the *i*-th power spectrum Sᵢ is calculated as a function of the values of the plurality *p* of filtered samples C₁, C₂, ... Cₚ.

The term "power spectrum" (also referred to as "power spectral density") means the pattern of the spectral powers of the frequency components of the digital signal composed of the plurality *p* of filtered samples C₁, C₂, ... Cₚ, wherein said frequency components are calculated by means of the discrete Fourier transform.

Advantageously, the power spectrum is calculated using the Lomb-Scargle algorithm.

From step 105 the method proceeds to step 106, wherein the *i*-th spectral power Pᵢ is calculated, as a function of the *i*-th power spectrum Sᵢ, in a frequency sub-interval, which is selected for example from the followings:
- low-frequency interval comprised between 0.04 Hz and 0.15 Hz, which is influenced by the sympathetic nervous system;
- high-frequency interval comprised between 0.15 Hz and 0.4 Hz, which is influenced by the parasympathetic nervous system.

In particular, the *i*-th spectral power Pᵢ is calculated as the integral of the *i*-th power spectrum Si in the considered frequency sub-interval.

From step 106 the method proceeds to step 107, wherein the value of the *i*-th spectral power Pᵢ is stored (into the memory 2-6).

From step 107 the method proceeds to step 108, wherein it is verified whether the total measurement time interval T_mt is terminated:
- in the negative case (i.e. the total measurement time interval T_mt is not terminated), the method proceeds with step 109;
- in the positive case (i.e. the total measurement time interval T_mt is terminated), the method proceeds with step 110.

In step 109 the next (i.e. the (i+1)-th) measurement time sub-interval Tᵢ is selected and the cycle composed of steps 102, 103, 104, 105, 106, 107 and 108 is repeated, until it is detected that the total measurement time interval T_mt is terminated (i.e. the sum of the values of the measurement time sub-intervals T₁, T₂, ... T_{K} is equal to the value of the total measurement time interval T_mt).

Therefore, the iteration of the above-described cycle generates a plurality K of spectral power values P₁, P₂, ... P_{K} and said values are stored into the memory 2-6, so as to be subsequently read for performing a processing.

For example, the value of the total measurement time interval T_mt is equal to 24 hours and thus it comprises a night-time phase wherein the subject being analyzed is asleep and a daytime phase wherein the subject is awake; in this case the plurality *p* of samples C₁, C₂, ... Cₚ defines a measurement time sub-interval T_mp which is a fraction of the total measurement time interval T_mt.

For example, considering T_mt= 24 hours, *p*=300 and 0% overlap between measurement time sub-intervals T_mp, the value of each *i*-th measurement time sub-interval T_mp is equal to about 5 minutes and thus every about 5 minutes the *i*-th spectral power Pᵢ is calculated and stored as indicated above in the previous steps 103-107, so that a plurality of about 288 values of the spectral power Pᵢ is obtained in the considered frequency sub-interval (K= 288, i=1, 2, ... 288).

Advantageously, in step 109, the (*i*+*1*)-th measurement time sub-interval is selected in such a way that the *(i*+*1)*-th measurement time sub-interval at least partly overlaps the *i*-th measurement time interval.

For example, the overlap percentage is comprised between 10% and 80%, in particular equal to 50%.

For example, considering *p*=300 and a 50% overlap, this means that the first 150 samples C₁, C₂, ... C₁₅₀ representative of the interbeat interval in the *(i*+*1)*-th measurement time sub-interval are equal to the last 150 samples C₁₅₁, C₁₅₂, ... C₃₀₀ representative of the interbeat interval in the *i*-th measurement time sub-interval, whilst the last 150 samples C₁₅₁, C₁₅₂, ... C₃₀₀ representative of the interbeat interval in the *(i*+*1)*-th measurement time sub-interval are different.

From step 108 the method proceeds to step 110, wherein a plurality K of values of the spectral power P₁, P₂, ... P_{K} is received (by reading from the memory 2-6) and said plurality K of values of the spectral power P₁, P₂, ... P_{K} is divided into a first and a second subset associated with a first and a second subset of the plurality *K* of measurement time intervals T₁, T₂, ... T_{K} respectively, wherein the first and second subsets of the plurality *K* of measurement time sub-intervals T₁, T₂, ... T_{K} are different and can be generated according to different criteria.

Considering Again the above example of the total measurement time interval T_mt= 24 hours, according to a first criterion the first subset of the plurality K of measurement time sub-intervals T₁, T₂, ... T_{K} comprises 16 daytime hours (for example from 6.00 AM to 11.00 p.m.), whilst the second subset of the plurality K of measurement time sub-intervals T₁, T₂, ... T_{K} comprises 8 night-time hours (for example from 11.00 p.m. to 6.00 AM).

A second criterion for the generation of the first and second subsets of the plurality K of measurement time sub-intervals T₁, T₂, ... T_{K} is selected based on parameters calculated as a function of signals detected by the portable electronic device 2.

For example, the first subset of the plurality K of measurement time sub-intervals T₁, T₂, ... T_{K} comprises ten minutes of time wherein the subject being analyzed is awake, whereas the second subset comprises ten minutes of time wherein the subject being analyzed is asleep and is snoring.

A third criterion for the generation of the first and second subsets of the plurality K of measurement time sub-intervals T₁, T₂, ... T_{K} is arbitrary.

For example, the first subset comprises two daytime hours and the second subset comprises two night-time hours.

From step 110 the method proceeds in parallel to steps 111 and 112:
- in step 111 a first statistical indicator I1 is calculated as a function of the values of the first subset of the plurality K of spectral power values P₁, P₂, ... P_{K};
- in step 112 a second statistical indicator I2 is calculated as a function of the values of the second subset of the plurality K of spectral power values P₁, P₂, ... P_{K}.

The first and the second statistical indicators are selected, for example, from the following:
- mean;
- mode;
- median.

Considering again the above example of the total measurement time interval T_mt = 24 hours and using the mean as the statistical indicator, the mean of about 192 spectral power values associated with the daytime phase is calculated, along with the mean of about 96 spectral power values associated with the night-time phase, so as to obtain a mean daytime value I1= M_{d} and a mean night-time value I2= Mₙ.

From steps 111 and 112 the method proceeds to step 113, wherein a result index I_res representative of the result of said processing of the acquired signal is calculated as a function of the values of the first and second statistical indicators: therefore the processing result signal S_es generated as output from the processing unit 2-2 is a function of the value of said result index I_res.

From step 113 the method proceeds to step 114, wherein the flow chart 100terminates.

Preferably, the result index I_res is the ratio between the second statistical indicator I2 and the first statistical indicator I1.

Preferably, in the example considered above, I_res is the ratio between the mean night-time Mₙ and the mean daytime M_{d} of the calculated spectral powers, i.e. I_res= Mₙ/M_{d}; in this case the processing unit 2-2 (in particular, the processing sub-module 2-2-.1) is configured to perform a comparison between said ratio and a threshold index value I_resₜₕ:
- if the value of said ratio Mₙ/M_{d} is greater than (or equal to) the threshold index value I_resₜₕ, the processing result signal S_es is representative of the possible presence of a sleep behaviour disorder, in particular of the RBD type;
- if the value of said ratio Mₙ/M_{d} is lower than the threshold index value I_resₜₕ, the processing result signal S_es is representative of the absence a sleep behaviour disorder, in particular of the RBD type.

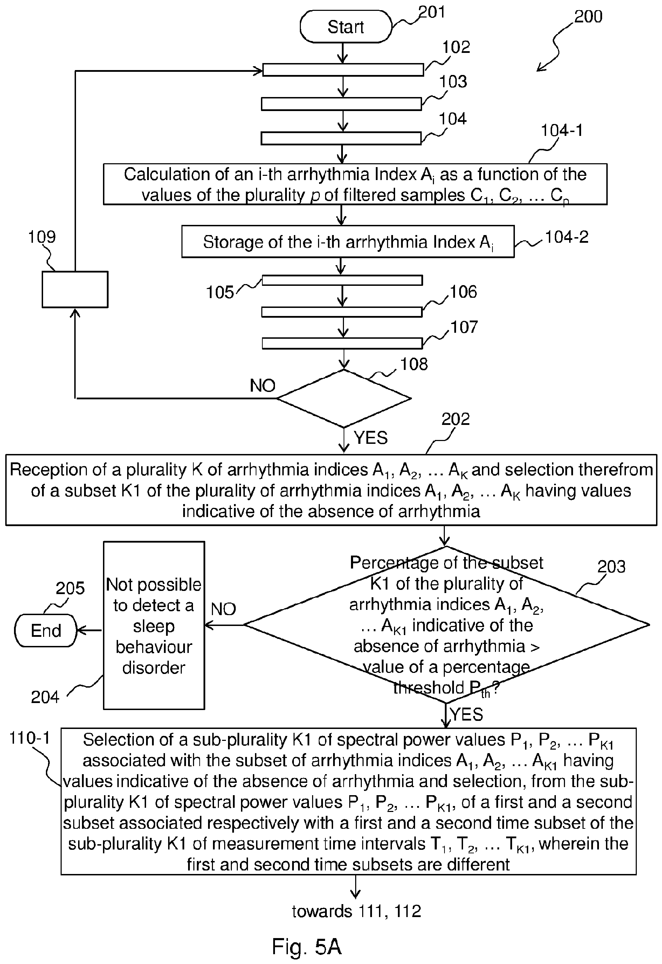

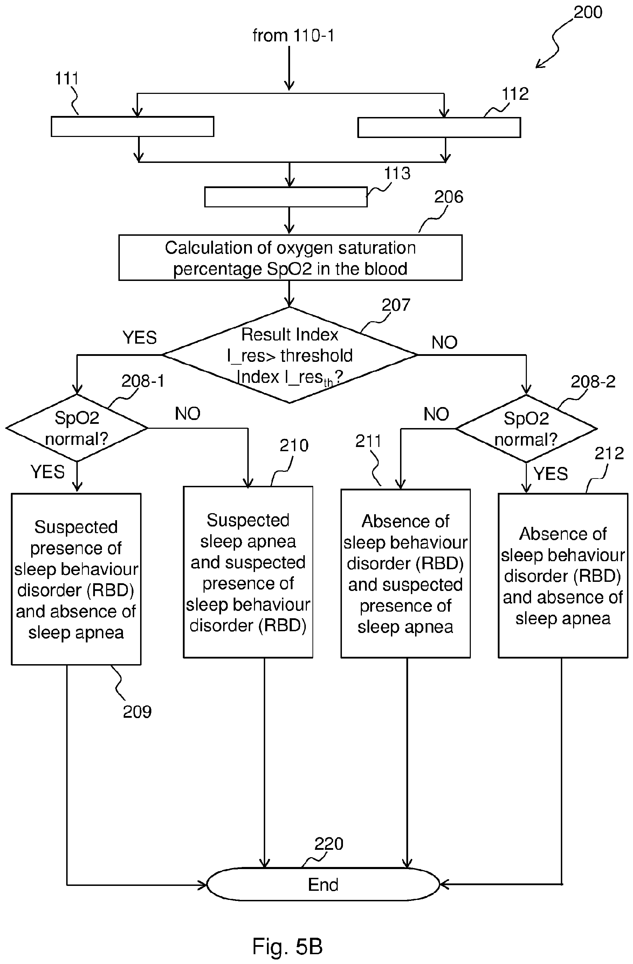

With reference to Figures 5A-5B, they show a flow chart 200 of the method for processing the signal S_V acquired from a living body of a subject according to a second embodiment of the invention.

The flow chart 200 is likewise implemented by means of a software program running on the processing unit 2-2, in particular in the processing module 2-2.2.

The processing method of the second embodiment allows to detect the presence of arrhythmias in the heartbeat of the subject being analyzed, so as to discard the measurements recorded in time intervals wherein said irregularities occurred.

Moreover, the processing method of the second embodiment allows to detect the presence of a suspected sleep apnea in the subject being analyzed.

The flow chart 200 comprises a part of the flow chart 100 and differs therefrom in the presence of the following additional steps.

The flow chart 200 starts with step 201 and proceeds with steps 102, 103 and 104, analogously to what was previously explained in relation to the flow chart 100 in Figure 4A.

From step 104 the method proceeds to step 104-1, wherein a *i*-th arrhythmia Index Aᵢ is calculated as a function of the values of the plurality *p* of filtered samples C₁, C₂, ... C_{P} representative of the interbeat interval.

For example, the *i*-th arrhythmia Index Aᵢ is a flag having a binary value having a value of 1 to indicate the presence of arrhythmia in the heart beat in the considered measurement time sub-interval Tᵢ (and thus that the corresponding plurality *p* of filtered samples C₁, C₂, ... Cₚ must be discarded), whilst it has a value of 0 to indicate the absence of arrhythmia of the heart beat in the considered measurement time sub-interval Tᵢ (and thus that the corresponding plurality *p* of filtered samples C₁, C₂, ... Cₚ should be considered for the purposes of subsequent processing).

Preferably, in step 104-1 it is calculated the value of the *i*-th standard deviation SDᵢ of the plurality p of filtered samples C₁, C₂, ... Cₚ representative of the interbeat interval in the considered measurement time sub-interval Tᵢ and it is verified whether the value of the *i*-th standard deviation SDᵢ is greater than or equal to a threshold value SDₜₕ (equal for example to 100):
- in the positive case (i.e. SDᵢ ≥ SDₜₕ), the value of the *i*-th arrhythmia Index Aᵢ is assigned equal to 1 to indicate the presence of arrhythmia in the considered measurement time sub-interval Tᵢ;
- in the negative case (i.e. SDᵢ < SDₜₕ), the value of the *i*-th arrhythmia Index Aᵢ is assigned equal to 0 to indicate the absence of arrhythmia in the considered measurement time sub-interval Tᵢ.

Alternatively, in step 104-1 it is calculated the value of the mean of the discrete derivative of the plurality p of filtered samples C₁, C₂, ... Cₚ in the considered measurement time sub-interval Tᵢ and it is verified whether the value of the *i*-th mean Mᵢ is greater than or equal to a threshold value Mₜₕ and thus the value of the *i*-th arrhythmia Index Aᵢ is calculated in a manner analogous to what was explained previously.

From step 104-1 the method proceeds to step 104-2, wherein the *i*-th arrhythmia Index Aᵢ is stored.

From step 104-2 the method proceeds to step 105 and subsequently to the previously illustrated steps 106, 107 and 108.

The cycle composed of the previous steps 102, 103, 104, 104-1, 104-2, 105, 106, 107 and 108 are repeated several times, until it is detected that the total measurement time interval T_mt is terminated.

From step 108 the method proceeds to step 202 if the total measurement time interval T_mt is terminated.

In step 202 a plurality K of arrhythmia indices A₁, A₂, ... A_{K} is received and a subset K1<K of the plurality of arrhythmia indices A₁, A₂, ... A_{K1} having values indicative of the absence of arrhythmia is selected.

From step 202 the method proceeds to step 203, wherein it is calculated the percentage of the subset K1 of the arrhythmia indices A₁, A₂, ... A_{K1} indicative of the absence of arrhythmia and it is verified whether the value of said percentage is greater than the value of a percentage threshold Pₜₕ (i.e. (K1/K)*100 is calculated):
- in the negative case, the method proceeds to step 204;
- in the positive case, the method proceeds to step 110-1.

In step 204 it is detected that the detection of the presence of a sleep behaviour disorder is not possible and then the flow chart 200 terminates at step 205.

Step 110-1 is analogous to step 110 of the flow chart 100 of the first embodiment of Fig.4A, the difference being that only a part of the plurality K of spectral power values P₁, P₂, ... P_{K} is taken into consideration (for the purposes of subsequent processing), i.e. a subset K1 of values (of the spectral power P₁, P₂, ... P_{K1}) associated with the subset of arrhythmia indices A₁, A₂, ... A_{K1} having values indicative of the absence of arrhythmias.

Therefore in step 110-1 it is selected a sub-plurality K1 of spectral power values P₁, P₂, ... P_{K1} associated with the subset of arrhythmia indices A₁, A₂, ... A_{K1} having values smaller than the value of the threshold arrhythmia Aₜₕ.

Moreover, in step 110-1 it is selected, from the sub-plurality K1 of spectral power values P₁, P₂, ... P_{K1}, a first and a second subset associated with a first and a second time subset of the sub-plurality K1 of measurement time intervals T₁, T₂, ... T_{K1} respectively , wherein the first and second time subsets are different.

From step 110-1 the method proceeds in parallel to steps 111, 112 and subsequently to step 113 wherein the result index I_res is calculated, in a manner analogous to what was illustrated previously for the flow chart 100 of the first embodiment of Fig.4A.

From step 113 the method proceeds to step 206 wherein the oxygen saturation percentage SpO2 in the blood is calculated.

From step 206 the method proceeds to step 207 wherein it is verified whether the value of the result index I_res is greater than a threshold index I_resₜₕ:
- in the positive case (i.e. I_res> I_resₜₕ), the method proceeds to step 208-1;
- in the negative case (i.e. I_res< I_resₜₕ), the method proceeds to step 208-2.

In step 208-1 it is verified whether the value of the oxygen saturation percentage SpO2 is normal:
- in the positive case, the method proceeds to step 209;
- in the negative case, the cycle proceeds to step 210.

In step 208-2 it is verified whether the value of the oxygen saturation percentage SpO2 is normal:
- in the positive case (i.e. I_res< I_resₜₕ), the method proceeds to step 212;
- in the negative case (i.e. I_res< I_resₜₕ), the method proceeds to step 211.

In step 209 it is detected a suspected presence of a sleep behaviour disorder (for example, of the RBD type) and an absence of sleep apnea and then the method proceeds to step 220 wherein the flow chart 200terminates.

Preferably, in step 209 the light-emitting diode of the electronic device 2 generates steady red light.

However, the red light switches on in a low-frequency intermittent mode (for example, 1/sec) if phenomenon 2 also occurs during the night.

The intermittent red light means that the result is more accurate.

Preferably, after step 209 a polysomnographic test should be performed to confirm the presence of the sleep behaviour disorder (for example, of the RBD type) in the subject being analyzed.

In step 210 it is detected a suspected presence of sleep apnea and a suspected presence of sleep behaviour disorder (for example, of the RBD type) in the subject being analyzed and then the method proceeds to step 220 wherein the flow chart 200 terminates.

Preferably, in step 210 the light-emitting diode of the electronic device 2 generates steady blue light.

If the presence of phenomenon 1 (which increases the probability of apnea) or of phenomenon 2 (which increases the probability of RBD) or both occur during the night, the blue light switches on in a slow intermittent mode (1/sec).

The intermittent blue light (low frequency, 1/sec) means that the result is more accurate.

Preferably, after step 210 a polysomnographic test should be performed to confirm the presence of sleep behaviour disorder (for example, of the RBD type) and/or the presence of sleep apnea in the subject being analyzed.

In step 211 it is detected an absence of sleep behaviour disorder (for example, of the RBD type) in the subject being analyzed and a suspected presence of sleep apnea and then the method proceeds to step 220 wherein the flow chart 200 terminates.

Preferably, in step 211 the light-emitting diode of the electronic device 2 generates steady yellow light.

If phenomenon 1 occurs as well during sleep, the light switches on in a low-frequency intermittent mode (1/sec).

The intermittent yellow light means that the result is more accurate.

Preferably, after step 211 cardiorespiratory polygraphy or a polysomnographic test should be performed to confirm the presence of sleep apnea in the subject being analyzed.

In step 212 it is detected an absence of sleep behaviour disorder (for example, of the RBD type) in the subject being analyzed and an absence of sleep apnea and then the method proceeds to step 220 wherein the flow chart 200 ends.

Preferably, in step 212 the light-emitting diode of the electronic device 2 generates intermittent green light.

The green light switches on in a low-frequency intermittent mode (1/sec) if, during the night, no single or repeated episodes of agitation, also accompanied by the emission of vocal sounds (shouts, moans, muttering, etc.), are recorded which last from 1 minute to 20 minutes, as detected by sensor 2-7 and 2-9.

The intermittent green light means that the result is accurate.

Advantageously, in steps 208-1 and 208-2 said verification of the oxygen saturation percentage is carried out according to the following steps:
- comparing the value of the oxygen saturation percentage with a threshold oxygen value SpO2ₜₕ for at least the duration of a first defined time interval (for example, for at least 10-15 seconds);
- repeating said comparison for a plurality of further first time intervals until reaching a second defined time interval which is smaller than or equal to the total measurement time interval T_mt (in particular, the second time interval is a multiple of the first time interval, for example an hour), thus generating a respective plurality of oxygen saturation events wherein the value of the oxygen saturation percentage is compared with the threshold oxygen value SpO2ₜₕ;
- calculating, during the second time interval, the number of said plurality of oxygen saturation events wherein the value of the oxygen saturation percentage is lower than the threshold oxygen value SpO2ₜₕ for at least the whole respective first defined time interval;
- if the calculated number is greater than or equal to an events threshold value Nₜₕ, detecting that the value of the oxygen saturation percentage SpO2 is critical and then the processing proceeds with a subsequent second time interval, until the total measurement time interval T_mt has elapsed;
- if the calculated number is lower than the events threshold value Nₜₕ, detecting that the value of the oxygen saturation percentage SpO2 is normal and then the processing proceeds with the subsequent second time interval, until the total measurement time interval T_mt has elapsed.

For example, the duration of the first defined time interval is comprised between 10 and 15 seconds, the duration of the second time interval is equal to one hour and it is verified whether the number of times during which the oxygen saturation percentage is lower than 90% (for at least 10-15 seconds) is greater than or equal to 5 in one hour:
- in the positive case, a suspected presence of sleep apnea is detected and thus a polysomnographic test is performed to confirm the presence of sleep apnea;
- in the negative case, the presence of sleep apnea is not detected and thus it is possible to proceed in order to detect a possible presence of sleep behaviour disorder (for example, RBD).

According to another example, it is verified whether the number of times during which the oxygen saturation percentage is lower than 90% (for at least 10-15 seconds) is greater than or equal to 15 during the night-time hours (for example between 10.00 PM and 6.00 AM).

The flow charts 100 and 200 of the first and second embodiments of the invention are implemented in the processing module 2-2.2, which is shown in greater detail in Figure 2B.

In particular, the processing module 2-2.2 comprises:
- a sub-module 2-2.2.1 configured to generate a plurality *p* of interbeat intervals (for example, calculated as a function of the times RR or NN);
- a sub-module 2-2.2.2 configured to calculate, as a function of the values of the plurality *p* of interbeat intervals and of the acceleration signal S_AC, the standard deviation (or another index of mean variability of the interbeat interval) in order to verify the presence of arrhythmias and configured to generate the *i*-th arrhythmia Index Aᵢ having binary values;
- a sub-module 2-2.2.3 configured to perform a verification of the plurality *p* of the interbeat intervals and to perform an adaptive filtering;
- a circular buffer 2-2.2.4 configured to store a plurality *p* of samples C₁, C₂, ... Cₚ representative of a respective plurality *p* of interbeat intervals, in particular a plurality *p* of times NN₁, NN₂, NN₃, ... NNₚ₋₁, NNₚ;
- a sub-module 2-2.2.5 configured to calculate an estimation of the power spectrum by means of a fast calculation of the periodogram, for example using the Lomb-Scargle algorithm;
- a sub-module 2-2.2.6 configured to calculate a vector 2-2.2.7 of dimension K of spectral powers in a frequency sub-interval, in particular a vector 2-2.2.7 of spectral powers LF₁, LF₂, LF₃, ... LF_{K-1}, LF_{K} in the low-frequency interval comprised between 0.04 Hz and 0.15 Hz, and moreover the sub-module 2-2.2.6 is configured to generate a vector 2-2.2.8 of dimension K of arrhythmia indices A₁, A₂, A₃, ... A_{K-1}, A_{K};
- a sub-module 2-2.2.9 configured to calculate the result index I_res as a function of the values of a first and second statistical indicator, in particular the ratio between the mean of the spectral powers in the low-frequency interval associated with the night-time and the mean of the spectral powers in the low-frequency interval associated with the daytime;
- a comparison sub-module 2-2.2.10 configured to perform a comparison between the result index I_res and a threshold index I_resₜₕ;
- a sub-module 2-2.2.11 configured to detect the absence of sleep behaviour disorder;
- a sub-module 2-2.2.12 configured to detect a suspected presence of sleep behaviour disorder.

The sub-module 2-2.2.6 is executed K times, so that at every *i*-th iteration (*i*= 1, 2, ... K) the *i*-th value of the spectral power LFᵢ is calculated.

Alternatively, the sub-module 2-2.2.6 is configured to calculate the vector 2-2.2.7 of spectral powers HF₁, HF₂, HF₃, ... HF_{K-1}, HF_{K} in the high-frequency interval comprised between 0.15 Hz and 0.4 Hz, and the sub-module 2-2.2.6 is also configured to generate the vector 2-2.2.8 of dimension K.

The operation of the portable electronic device 2 will now be described, also making reference to figures 1, 2 and 4A-4B.

The following assumptions are considered:
- the portable electronic device 2 has an external shape as shown in Figure 3, i.e. a main body 2a with a disk-shaped casing, an internal photoplethysmographic sensor 2-1 and an external one 2-10 with a clip-on unit and an electrical connection cable between the external sensor 2-10 and the main body 2a;
- the mobile electronic device 3 is a smartphone running a software application;
- the total measurement time interval T_mt is equal to 24 hours;
- *p*>=300, i.e. for every *i*-th measurement time sub-interval Tᵢ it is calculated at least 300 values of the samples C₁, C₂, ... C₃₀₀ representative of at least 300 interbeat intervals of the heart of the subject being analyzed;
- the value of the measurement time sub-intervals T₁, T₂, ... T_{K} is equal to 5 minutes and the overlap between adjacent measurement time sub-intervals T₁, T₂, ... T_{K} is equal to 0%, thus K ≥ 288;
- the daytime hours are comprised between 6.00 AM and 10.00 PM, whilst the night-time hours are comprised between 10.00 PM and 6.00 AM;
- the considered frequency sub-interval is composed of low frequencies ranging between 0.04 Hz and 0.15 Hz;
- the first and second statistical indicators are the mean;
- neither the accelerometer 2-7, nor the microphone 2-9, nor the temperature sensor 2-8 are present.

At 6.00 AM the subject to be analyzed is at home and wakes up at 6.00 AM, then puts on the portable electronic device 2 by securing it to his earlobe by means of the clip-on unit and then switches on the portable electronic device 2 by pressing the button 2c.

Subsequently, in the time interval comprised between 6.00 AM and 10:00 PM the subject being analyzed engages in normal everyday activities (for example, work), continuing to wear the portable electronic device 2 secured to his ear.

In particular, in the first measurement time sub-interval T₁ comprised between 6.00 AM and 6.05 AM (excluded) the processing unit 2-2 of the portable electronic device 2 performs the steps 102, 103, 104, 105, 106 and 107 of the flow chart 100 and then it calculates at least 300 values of the samples C₁, C₂, ... C₃₀₀, ..., representative of at least 300 interbeat intervals of the heart of the subject being analyzed, then it calculates the first power spectrum S₁ and the first spectral power P₁, thus it stores the value of the first spectral power P₁ into the memory 2-6.

At the time 6.05 AM the processing unit 2-2 performs step 108 and detects that the total measurement time interval T_mt is not terminated, because 5 minutes out of a total of 24 hours have elapsed.

Therefore at time 6.05 AM the processing unit 2-2 performs the step 109 wherein it selects the second measurement time sub-interval T₂ comprised between 6.05 AM and 6.10 AM (excluded), then again it performs steps 102, 103, 104, 105, 106 and 107 of the flow chart 100, again calculating 300 values of the samples C₁, C₂, ... C₃₀₀,..., representative of at least 300 interbeat intervals of the heart of the subject being analyzed, then it calculates the second power spectrum S₂ and the second spectral power P₂ and finally it stores the value of the second spectral power P₂ into the memory 2-6.

At time instants comprised between 6.10 AM and 10:00 PM (excludied) the processing unit 2-2 executes the cycle comprising steps 102, 103, 104, 105, 106, 107, 108 and 109 for further 190 times at most, thus generating at the time 10:00 PM a total of at most 192 spectral power values P₁, P₂, ... P₁₉₂.

At 10.00 PM the subject being analyzed goes to sleep, continuing to wear the portable electronic device 2 secured to his or her ear, until 6.00 AM.

In the measurement time sub-interval T₁₉₃ comprised between 10.00 PM and 10.05 PM (excluded) the processing unit 2-2 of the portable electronic device 2 carries out the steps 102, 103, 104, 105, 106 and 107 of the flow chart 100 and then it calculates at least 300 values of the samples C₁, C₂, ... C₃₀₀ representative of at least 300 interbeat intervals of the heart of the subject being analyzed, then it calculates the power spectrum S₁₉₃ and the spectral power P₁₉₃, this it stores the value of the spectral power P₁₉₃ into the memory 2-6.

At the time 10.05 PM the processing unit 2-2 performs the step 108 and it detects that the total measurement time interval T_mt is not terminated, because 16 hours and 5 minutes out of a total of 24 hours have elapsed.

Therefore at time 10.05 PM the processing unit 2-2 performs the step 109 wherein it selects the measurement time sub-interval T₁₉₄ comprised between 10.05 PM and 10.10 PM (excluded), then again carries out steps 102, 103, 104, 105, 106 and 107 of the flow chart 100, again calculates at least 300 values of the samples C₁, C₂, ... C₃₀₀ representative of at least 300 interbeat intervals of the heart of the subject being analyzed, then calculates the power spectrum S₁₉₄ and the spectral power P₁₉₄, and finally stored the value of the spectral power P₁₉₄ in the memory 2-6.

At time instants comprised between 10.10 PM and 6.00 AM (excluded) the processing unit 2-2 executes the cycle comprising steps 102, 103, 104, 105, 106, 107, 108 and 109 for further 94 times, thus generating at the time 6.00 AM a total of at most further 96 spectral power values P₁₉₃, P₁₉₄, ... P₂₈₈.

At the time 6.00 AM the processing unit 2-2 performs the step 108 and detects that the total measurement time interval T_mt is terminated, because 24 hours have elapsed.

Subsequently, the processing unit 2-2 performs the step 110 wherein it receives 288 values of the spectral power P₁, P₂, ... P₂₈₈, then it divides the first 192 values P₁, P₂, ... P₁₉₂ into a first sub-time interval comprising the daytime interval comprised between 6.00 AM and 10.00 PM and the second 96 values P₁₉₃, P₁₉₄, ... P₂₈₈ into a second sub-time interval comprising the night-time interval comprised between 10.00 PM and 6.00 AM.

Subsequently, the processing unit 2-2 performs the step 111 wherein it calculates the daytime mean M_{d} of the first 192 values P₁, P₂, ... P₁₉₂ and performs the step 112 wherein it calculates the night-time mean Mₙ of the second 96 values P₁₉₃, P₁₉₄, ... P₂₈₈.

Subsequently, the processing unit 2-2 performs the step 113 wherein it calculates the result index I_res equal to the ratio between the night-time mean Mₙ and the daytime mean M_{d} , i.e. I_res= Mₙ/M_{d}, and detects that the value of said ratio Mₙ/M_{d} is greater than the value of the threshold index I_resₜₕ: in this case it is detected a suspected presence of sleep behaviour disorder of the RBD type (for example, a red light is generated by the light-emitting diode mounted on the portable electronic device 2) and thus the subject being analyzed takes into account whether he should undergo a polysomnographic test to confirm the presence of RBD.

Advantageously, the portable electronic device 2 is equipped with a self-evaluation system based on the light-emitting diode 2-11 with different colours, which light up in a steady or intermittent mode.

Depending on the colour and/or intermittence, the subject can easily obtain (without a physician's aid) conclusive information about the possible presence or absence of RBD and/or apnea, according to the following Table.

**Table for self-evaluation of the results obtainable with the portable electronic device 2**

| Light of 2-11 | information | conclusions |
|---|---|---|
| intermittent green * | Result index I_res with value <I_resₜₕ + normal oxygen saturation percentage + absence of single or repeated episodes of agitation with vocal emissions (shouts, moans, muttering, etc.) lasting from 1 to 20 minutes. | normal |
| | | |
| steady yellow | Result index I_res with value < I_resₜₕ + reduced oxygen saturation percentage | suspected apnea |
| intermittent yellow* | Result index I_res with value < I_resₜₕ + reduced oxygen saturation percentage + termination of snoring in concomitance with reduction in oxygen saturation | suspected apnea |
| | | |
| steady blue | Result index I_res with value ≥ I_resₜₕ + reduced oxygen saturation percentage | suspected apnea + suspicion of RBD |
| intermittent blue* | Result index I_res with value ≥ I_resₜₕ + reduced oxygen saturation percentage + termination of snoring in concomitance with reduction of oxygen saturation and/or presence of simultaneous agitation with vocal emissions (shouts, moans, muttering, etc.) lasting from 1 to 20 minutes. | suspected apnea + suspicion of RBD |
| | | |
| steady red | Result index I_res with value ≥ I_resₜₕ + normal oxygen saturation percentage | suspicion of RBD |
| intermittent red * | Result index I_res with value ≥ I_resₜₕ + normal oxygen saturation percentage + simultaneous presence of single or repeated episodes of agitation with emission of vocal sounds (shouts, moans, muttering, etc.) lasting from 1 to 20 minutes. | suspicion of RBD |

It should be noted that the intermittent light (indicated with an asterisk*) represents a result with a higher level of reliability than a steady light of the same colour.

The processing unit 2-2 is thus configured to generate the driving signal S_drv having suitable values such to switch on the light-emitting diode 2-11, as indicated in the Table above.

The method is applicable to subjects who, upon reawakening, are aware of having slept during the night; if the subject is aware of not having slept, the test must be repeated.

Preferably, according to a variant of the invention the portable electronic device 2 belongs to a multi-function modular system which allows to implement different configurations, based on a pre-established objective.

Therefore said modular system comprises the mobile electronic device 3, the portable electronic device 2 and one or more body detectors connected to the mobile electronic device 3 by means of short-range wireless signals (for example, of the Bluetooth, WiFi or ZigBee type).

The set of the mobile electronic device 3, of the portable electronic device 2 and of the body detectors make up a network of body sensors (Body Sensor Area Network, abbreviated BSAN), wherein the mobile electronic device 3 operates as the central node of the network itself.

Said body detectors are selected from at least one of the following list:
- electrodes for electromyography (EMG);
- electrodes for the acquisition of an electrocardiogram (ECG);
- chest belt and/or abdominal belt for measuring breathing acts;
- nasal thermocouple for measuring the inhaled/exhaled air flow;
- accelerometers applied with straps on the upper and lower limbs to detect movements;
- a pneumatically actuated device for measuring arterial pressure which may be applied on the arm or wrist;
- three-channel micro polygraph for electroencephalograms (EEG), electrooculograms (EOG) and electromyography (EMG).

The following configurations of the modular multi-function system are possible:
1) mobile electronic device 3, portable electronic device 2 and wireless device with electrodes for electromyography;
2) mobile electronic device 3, portable electronic device 2 and the following further devices connected to the wireless mobile electronic device 3: device with electrodes for electromyography, devices with chest belt and/or abdominal belt, device for measuring arterial pressure;
3) mobile electronic device 3, portable electronic device 2, further wireless devices of configuration 2) and the following additional devices connected via a wireless connection to the wireless mobile electronic device 3: three-channel micro polygraph for electroencephalograms and electrooculograms;
4) mobile electronic device 3, portable electronic device 2 and further devices of configuration 3), without the analysis of sleep apnea;
5) mobile electronic device 3, portable electronic device 2 and any combination of the following further devices connected to the wireless mobile electronic device 3: device with electrodes for electromyography, device with electrodes for the acquisition of an electrocardiogram, micro polygraph with channels for electroencephalograms and electrooculograms, devices with chest belt and/or abdominal belt, device for measuring arterial pressure, device with accelerometers applied with straps on the upper and lower limbs to detect movements, device with a nasal thermocouple for measuring the inhaled/exhaled air flow.

Configuration 1) improves the accuracy of the calculation for identifying RBD because it detects the presence of atony or muscular activity connected to sleep. This configuration ensures the automatic detection of episodes of muscular atony that occur during sleep. Each episode of atony is considered as true if it lasts at least one minute. The system detects all the episodes of atony that occur during the night and determines the time duration of each single episode. Atony is considered a reduction in the energy of the electromyographic signal by a predetermined percentage amount. This configuration allows to increase the accuracy of interpretation of the data obtained from the basic configuration. Preferably, if episodes of atony occur during the night, the light-emitting diode of the portable electronic device 2 will switch on in the high-frequency intermittent mode (2-4/sec) so as to provide a better accuracy of interpretation of the data based on the following scheme:
- high-frequency intermittent green light: indicates the presence of atony and a normal percentage of oxygen saturation in the blood;
- high-frequency intermittent yellow light: indicates the presence of atony and a low percentage of oxygen saturation in the blood.

In the event of absence of atony, the high-frequency intermittent red light indicates a high index and a normal percentage of oxygen saturation in the blood, whereas a high-frequency intermittent blue light indicates a high index and a low percentage of oxygen saturation in the blood.

Moreover, this simple configuration does not require the intervention of technical personnel, it can be installed and managed by the patient, is particularly useful for screening activities, and does not create any obstacle to falling asleep and maintaining a qualitatively and quantitatively optimal sleep. This configuration requires specialist medical assistance in interpreting the results in the absence of atony.

Configuration 2) is particularly useful for the detailed study of apnea in subjects with suspected sleep apnea based on the data acquired with the devices 2 and 3 of configuration 1).

Configuration 2) has the advantage of being compact in size and of not requiring the presence of cables for connecting to the base unit, which are typical of traditional polygraphs, and does not interfere with falling asleep or maintaining a qualitatively and quantitatively optimal sleep.

Configuration 3) extends the portable electronic device 2 to a medical instrument with the same diagnostic potential of a traditional polysomnograph, requires the presence of medical and technical personnel for its application, has a more compact size, does not require the presence of cables for connecting to the base unit, which are typical of traditional polygraphs, and interferes less with falling asleep and maintaining a qualitatively and quantitatively optimal sleep.

Configuration 4) is dedicated exclusively to the study and clinical diagnosis of RBD, and requires the intervention of medical and technical personnel.

Configuration 2) requires medical assistance for interpreting the data, but does not require technical assistance for applying the devices.

Configurations 3), 4) and 5) require technical assistance for applying the devices and specialist medical advice for interpreting the data; in these configurations the system assures the correlation of different variables that are useful for the purpose of interpreting the phenomena (example: pairing of O2 saturation and respiratory function detected by the belts and the nasal thermocouple).

It is also an object of the present invention a method for processing a signal acquired from a living body.

The processing method comprises the steps of:
a) receiving an acquisition signal representative of a variation in volume of the blood flow of a human tissue of the living body;
b) calculating, in a defined measurement time sub-interval and as a function of the values of the acquisition signal, a plurality of samples C₁, C₂, ... Cₚ representative of a respective plurality of interbeat intervals between adjacent heartbeats;
c) calculating, as a function of the values of the plurality of samples, a power spectrum in a frequency interval;
d) calculating, as a function of said power spectrum, a spectral power Pᵢ in a frequency sub-interval;
e) repeating the steps b), c) and d) for a plurality of further measurement time sub-intervals belonging to a total measurement time interval T_mt, so as to obtain a plurality of spectral power values P₁, P₂, ... P_{K} associated with the sub-plurality of measurement time intervals;
f) calculating a first statistical indicator as a function of a first subset of the plurality of spectral power values associated with a first subset of the plurality of measurement time sub-intervals;
g) calculating a second statistical indicator as a function of a second subset of the plurality of spectral power values associated with a second subset of the plurality of measurement time sub-intervals, wherein the first and second subsets of the plurality of measurement time sub-intervals are different;
h) generating, as a function of the values of the first and second statistical indicators, a result index representative of the result of said processing of the acquired signal.

Steps b)-h) of the processing method can be implemented on the processing unit 2-2 of the portable electronic device 2, or they can be implemented on a processing unit inside the mobile electronic device 3.

It is also an object of the present invention a computer program comprising software code portions executed on the processing unit 2-2 of the portable electronic device 2, in particular in the processing module 2-2.2; alternatively, the program is executed on a processing unit inside the mobile electronic device 3.

The software program performs the steps of the above-described method for processing the signal acquired from a living body.

## Claims

1. Electronic device (2) for the automatic detection of behaviour disorders during the REM sleep phase and sleep apnea, the device (2) comprising:
- an internal sensor (2-1) configured to be applied in contact with or in proximity to a tissue of a living body and to generate an acquisition signal (S_V) representative of a variation in the volume of the blood flow of a human tissue of the living body;
- an electronic output device (2-11);
- a processing unit (2-2) **characterized in that** the processing unit is configured to:
• receive (102; 2-2.2.1) the acquisition signal (S_V) and calculate (103; 2-2.2.4), in a defined measurement time sub-interval (Tᵢ) and as a function of the values of the acquisition signal (S_V), a plurality of samples (C₁, C₂, ... Cₚ; NN₁, NN₂,... NNₚ) representative of a respective plurality of interbeat intervals between adjacent heartbeats;
• calculate (105; 2-2.2.5), as a function of the values of the plurality of samples, a power spectrum (Sᵢ) in a frequency interval;
• calculate (106; 2-2.2.6), as a function of said power spectrum (Si), a spectral power (Pᵢ) in a frequency sub-interval;
• repeat (108, 109, 110) said calculation of the plurality of samples, of the respective power spectrum and of the respective spectral power for a plurality of further measurement time sub-intervals (T₁, T₂, ... T_{K}) belonging to a total measurement time interval (T_mt), so as to obtain a plurality of spectral power values (P₁, P₂, ... P_{K}) associated with the plurality of measurement time sub-intervals (T₁, T₂, ... T_{K});
• calculate a first statistical indicator (11) selected from the mean, the mode and median of a first subset of the plurality of spectral power values associated with a first subset of the plurality of measurement time sub-intervals;
• calculate a second statistical indicator (12) selected from the mean, the mode and the median of a second subset of the plurality of spectral power values associated with a second subset of the plurality of measurement time sub-intervals, wherein the first and second subsets of the plurality of measurement time sub-intervals are different;
• calculate a result index (I_res) that is the ratio between the second statistical indicator (12) and the first statistical indicator (I1).

2. Device according to claim 1, the device further comprising an electronic output device (2-11), wherein the processing unit is further configured to:
• generate, as a function of the acquisition signal, an oxygen saturation signal representative of the oxygen saturation percentage in the blood;
• compare the value of the oxygen saturation percentage with respect to a threshold oxygen value for at least the duration of a first defined time interval, in particular comprised between 10 seconds and 15 seconds;
• repeat said comparison for a plurality of further first time intervals until reaching a second defined time interval which is smaller than or equal to the total measurement time interval (T_mt), the second time interval being equal, in particular, to 1 hour, thus generating a respective plurality of oxygen saturation events wherein the value of the oxygen saturation percentage is compared with the threshold oxygen value;
• calculate, during the second time interval, the number of said plurality of oxygen saturation events wherein the value of the oxygen saturation percentage is lower than the threshold oxygen value for at least the whole respective first defined time interval;
• if said calculated number is greater than or equal to an events threshold value, detect a critical value of the oxygen saturation percentage;
• if said calculated number is lower than the events threshold value, detect a normal value of the oxygen saturation percentage;
• generate, as a function of the value of the result index (I_res) and of the detected value of the oxygen saturation percentage, a driving signal (S_drv) of the electronic output device (2-11).

3. Device (2) according to any one of the previous claims, wherein the processing unit is further configured to:
• calculate (104-1) an arrhythmia index as a function of the values of the plurality of the filtered samples representative of the interbeat interval;
• repeat (102, 103, 104, 104-1, 104-2, 105, 106, 107, 108, 109) the calculation of the plurality of samples, of the respective power spectrum and of the spectral power for the plurality of the further measurement sub-intervals, so as to obtain the plurality of spectral power values and a corresponding plurality of values of the arrhythmia index (A₁, A₂, ... A_{K});
• select (202) a subset of the plurality of values of the arrhythmia index having values indicative of the absence of arrhythmia;
• select a sub-plurality of spectral power values associated with said selected subset defining a percentage greater than a percentage threshold;
• select the first and second subsets of the plurality of spectral power values from the selected sub-plurality of values of the spectral power.

4. Device (2) according to any one of the previous claims, further comprising a accelerometer with at least one axis (2-7) configured to generate an acceleration signal (S_AC) representative of the acceleration value of the electronic device along the at least one axis,
wherein the processing unit (2-2) is further configured to receive the acceleration signal (S_AC) and perform, as a function thereof, an adaptive filtering of at least a part of the values of the plurality of calculated samples, wherein the processing unit is further configured to:
• calculate, as a function of the values of the acceleration signal, an energy index representative of the energy on the at least one axis in at least one time interval comprised between two adjacent heartbeats;
• filter, as a function of the comparison between the value of the calculated energy index and an energy threshold, at least a part of the values of the plurality of calculated samples.

5. Device (2) according to the previous claim, wherein the processing unit (2-2) is further configured to:
- compare the value of the energy index with respect to the threshold value for a third time interval;
- generate, as a function of the value of the result index and of said energy comparison, the driving signal (S_drv) of the electronic output device (2-11).

6. Device (2) according to the previous claim, wherein the processing unit is further configured to generate, as a function of the value of the result index (I_res), of said comparison of energy and of the detected value of the oxygen saturation percentage, the driving signal (S_drv) of the electronic output device (2-11).

7. Device (2) according to claims 5 or 6, further comprising a microphone (2-9) configured to generate an audio signal (S_AD) representative of snoring, wherein the processing unit (2-2) is further configured to:
- process the audio signal and detect therefrom the presence of snoring;
- generate, as a function of the value of the result index (I_res), of the detected value of the oxygen saturation percentage and of the value of the audio signal (S_AD) representative of snoring, the driving signal (S_drv) of the electronic output device (2-11).

8. Device (2) according to claims 5, 6 or 7, further comprising a microphone (2-9) configured to generate the audio signal (S_AD) further representative of an emission of vocal sounds, wherein the processing unit (2-2) is further configured to:
- process the audio signal (S_AD) and detect therefrom the presence of the vocal sounds;
- generate, as a function of the value of the result index (I_res), of said energy comparison, of the detected value of the oxygen saturation percentage and of the value of the audio signal (S_AD) representative of the emission of vocal sounds and/or of snoring, the driving signal (S_drv) of the electronic output device (2-11).

9. Device (2) according to any one of the previous claims, wherein:
- said frequency interval is comprised, alternatively, between 0.04 Hz and 0.15 Hz or between 0.15 Hz and 0.4 Hz;
- the measurement time sub-interval is at least 5 minutes, in particular equal to 10 minutes;
- the sum of the plurality of measurement time sub-intervals comprises at least 24 hours, wherein the first subset comprises at least a part of the daytime interval wherein the subject to be analyzed is awake and the second subset comprises at least a part of the night-time interval wherein the subject is asleep, or vice-versa.

10. Device (2) according to any one of the previous claims, wherein two adjacent measurement time sub-intervals at least partly overlap by a percentage comprised between 10% and 80%, in particular equal to 50%.

11. Device (2) according to any one of the previous claims, wherein the processing unit (2-2) is further configured to filter, in said measurement time sub-interval, at least a part of the values of the plurality of calculated samples, as a function of the comparison between the values of the plurality of calculated samples and the following values:
• an interval of values comprised between a minimum interbeat value and a maximum interbeat value; and/or
• a maximum value of variation between adjacent inter-beats.

12. Device (2) according to any one of the previous claims, wherein:
- the device (2) has a casing having a substantially circular shape with a diameter comprised between 20 mm and 30 mm;
- said sensor (2-1) is a plethysmographic sensor, in particular a photoplethysmographic sensor, of the reflectance type, positioned inside a main body (2a) of the casing and configured to generate a plethysmographic signal indicative of electrical potentials generated on the human tissue on which said sensor is applied as a result of cardiac cellular activity;
the device (2) further comprising a sensor (2-10) external to the main body (2a) and a connection cable (2b) for connecting the external sensor (2-10) to the main body (2a), the external sensor (2-10) being enclosed in an ear clip-on unit,
wherein the external sensor (2-10) is of the reflectance or penetrating type, the processing unit (2-2) being configured to detect the connection of the external sensor (2-10) and to disable therefrom the operation of the internal sensor (2-1).

13. Electronic system (1) comprising:
- an electronic device (2) according to any one of the previous claims, the electronic device further comprising a transceiver (2-3) of wireless signals configured to generate a short-range wireless signal (S_r_sd) carrying data acquired or processed by the electronic device (2);
- a mobile electronic device (3) comprising a screen and configured to:
• receive said short-range wireless signal (S_r_sd) carrying said acquired or processed data;
• process the acquired data;
• display the processed received data and/or the processed acquired data on the screen.

14. Electronic system (1) according to the previous claim, further comprising at least one further body detector (4, 5, 6) connected to the mobile electronic device (3) by means of respective short-range wireless signals.

15. Electronic system (1) according to the previous claim, comprising a plurality of body detectors (4, 5, 6) selected from the following:
- electrodes for electromyography;
- electrodes for the acquisition of an electrocardiogram;
- chest belt and/or abdominal belt for measuring breathing acts;
- nasal thermocouple for measuring the inhaled/exhaled air flow;
- accelerometers applied with straps on the upper and lower limbs to detect movements;
- pneumatically actuated device for measuring arterial pressure which may be applied on the arm or wrist;
- three-channels micro-polygraph for electroencephalograms, electrooculograms and electromyography.

16. Method for processing a signal (S_V) representative of a variation in volume of the blood flow of a human tissue of the living body using the electronic device (2) according to claims 1 to 12, comprising the steps of:
b) calculating (103), in a defined measurement time sub-interval (Tᵢ) and as a function of the values of the acquisition signal (S_V), a plurality of samples (C₁, C₂, ... Cₚ; NN₁, NN₂,... NNₚ) representative of a respective plurality of interbeat intervals between adjacent heartbeats;
c) calculating (105), as a function of the values of the plurality of samples, a power spectrum (Sᵢ) in a frequency interval;
d) calculating (106), as a function of said power spectrum (Sᵢ), a spectral power (Pᵢ) in a frequency sub-interval;
e) repeating the steps b), c) and d) for a plurality of further measurement time sub-intervals (T₁, T₂, ... T_{K}) belonging to a total measurement time interval (T_mt), so as to obtain a plurality of spectral power values (P₁, P₂, ... P_{K}) associated with the sub-plurality of measurement time intervals (T₁, T₂, ... T_{K});
f) calculating (111) a first statistical indicator (11) selected from the mean, the mode and median of a first subset of the plurality of spectral power values associated with a first subset of the plurality of measurement time sub-intervals;
g) calculating (112) a second statistical indicator (12) selected from the mean, the mode and median of a second subset of the plurality of spectral power values associated with a second subset of the plurality of measurement time sub-intervals, wherein the first and second subsets of the plurality of measurement time sub-intervals are different;
h) calculating (113) a result index (I_res) that is the ratio between the second statistical indicator (12) and the first statistical indicator (I1)

17. Processing method according to claim 16, further comprising, before step h), the step of generating (206), as a function of the acquisition signal, an oxygen saturation signal representative of the oxygen saturation percentage in the blood,
the method further comprising, after step h), the steps of:
i) comparing the value of the oxygen saturation percentage with respect to a threshold oxygen value for at least the duration of a first defined time interval, in particular comprised between 10 seconds and 15 seconds;
j) repeating step i) for a plurality of further first time intervals until reaching a second defined time interval which is smaller than or equal to the total measurement time (T_mt) interval, in particular the second time interval being equal to 1 hour, thus generating a respective plurality of oxygen saturation events wherein the value of the oxygen saturation percentage is compared with the threshold oxygen value;
k) calculating, during the second time interval, the number of said plurality of oxygen saturation events wherein the value of the oxygen saturation percentage is lower than the threshold oxygen value for at least the whole respective first defined time interval;
I) if said calculated number is greater than or equal to an events threshold value, detecting a critical value of the oxygen saturation percentage;
m) if said calculated number is lower than the events threshold value, detecting a normal value of the oxygen saturation percentage;
n) generating, as a function of the value of the result index (I_res) and of the detected value the oxygen saturation percentage, a driving signal (S_drv) of an electronic output device (2-11).

18. Method according to claims 16 or 17, further comprising, before step c), step c1) of calculating (104-1) an arrhythmia index as a function of the values of the plurality of the filtered samples representative of the interbeat interval,
the method comprising repeating steps b), c) and d) for the plurality of the further measurement sub-intervals, so as to obtain the plurality of spectral power values and a corresponding plurality of values of the arrhythmia index (A₁, A₂, ... A_{K}),
the method further comprising:
- selecting (202) a subset of the plurality of values of the arrhythmia index having values indicative of the absence of arrhythmia;
- selecting (203) a sub-plurality of spectral power values associated with said subset selected defining a percentage greater than a percentage threshold;
- selecting (110-1) the first and second subsets of the plurality of spectral power values from the selected sub-plurality of values of the spectral power.

19. Method according to any one of claims 16 to 18, further comprising:
- receiving an acceleration signal (S_AC) representative of the value of acceleration along at least one axis (2-7) and calculating therefrom an energy index representative of the energy on at least one axis in at least one time interval comprised between two adjacent heartbeats;
- comparing the value of the energy index with respect to the threshold value for a third time interval;
- generating, as a function of the value of the result index and of said energy comparison, the driving signal (S_drv) of the electronic output device (2-11).

20. Method according to the previous claim, wherein the generating step comprises generating, as a function of the value of the result index (I_res), of said energy comparison and of the detected value of the oxygen saturation percentage, the driving signal (S_drv) of the electronic output device (2-11).

21. Method according to claims 19 or 20, further comprising:
- receiving an audio signal (S_AD) representative of snoring;
- processing the audio signal (S_AD) and detecting therefrom the presence of snoring;
- generating, as a function of the value of the result index (I_res), of the detected value of the oxygen saturation percentage and of the value of the audio signal (S_AD) representative of snoring, the driving signal (S_drv) of the electronic output device (2-11).

22. Method according to claims 19, 20 or 21, further comprising:
- receiving an audio signal (S_AD) representative of the emission of vocal sounds;
- processing the audio signal (S_AD) and detecting therefrom the presence of the vocal sounds;
- generating, as a function of the value of the result index (I_res), of said energy comparison, of the detected value of the oxygen saturation percentage and of the value of the audio signal (S_AD) representative of the emission of vocal sounds and/or snoring, the driving signal (S_drv) of the electronic output device (2-11).

23. Method according to claim 16 to 22, wherein
- said frequency interval is comprised, alternatively, between 0.04 Hz and 0.15 Hz or between 0.15 Hz and 0.4 Hz;
- the measurement time sub-interval is at least 5 minutes, in particular equal to 10 minutes;
- the value of the total measurement time interval (T_mt) is equal to 24 hours and comprises a night-time phase wherein the subject being analyzed is asleep and a daytime phase wherein the subject is awake.

24. Computer program comprising software code portions adapted to perform the steps of the method according to claims 16-23, when said program is run on at least one computer.

## Patentansprüche

1. Elektronische Vorrichtung (2) zur automatischen Erkennung von Verhaltensstörungen während der REM-Schlafphase und von Schlafapnoe, wobei die Vorrichtung (2) umfasst:
- einen internen Sensor (2-1), der ausgelegt ist, um in Kontakt mit oder in der Nähe von einem Gewebe eines lebenden Körpers angebracht zu werden und um ein Erfassungssignal (S_V) zu generieren, das repräsentativ für eine Veränderung im Volumen des Blutstroms eines menschlichen Gewebes des lebenden Körpers ist;
- eine elektronische Ausgabevorrichtung (2-11);
- eine Verarbeitungseinheit (2-2), **dadurch gekennzeichnet, dass** die Verarbeitungseinheit ausgelegt ist, um
• das Erfassungssignal (S_V) zu empfangen (102; 2-2.2.1) und eine Vielzahl an Proben (C₁, C₂, ... Cₚ; NN₁, NN₂, ... NNₚ), die repräsentativ für eine jeweilige Vielzahl an Interbeat-Intervallen zwischen angrenzenden Herzschlägen sind, in einem vorgegebenen Messzeitunterintervall (Tᵢ) und als eine Funktion der Werte des Erfassungssignals (S_V) zu berechnen (103; 2-2.2.4);
• als eine Funktion der Werte der Vielzahl an Proben ein Leistungsspektrum (Sᵢ) in einem Frequenzintervall zu berechnen (105; 2-2.2.5);
• als eine Funktion des Leistungsspektrums (Sᵢ) eine spektrale Leistung (Pᵢ) in einem Frequenzsubintervall zu berechnen (106; 2-2.2.6);
• die Berechnung der Vielzahl an Proben des jeweiligen Leistungsspektrums und der jeweiligen spektralen Leistung für eine Vielzahl an weiteren Messzeitsubintervallen (T₁, T₂, ... T_{K}) zu wiederholen (108, 109, 110), die einem Gesamtmesszeitintervall (T_mt) angehören, sodass eine Vielzahl an spektralen Leistungswerten (P₁, P₂, ... P_{K}) erhalten wird, assoziiert mit der Vielzahl an Messzeitsubintervallen (T₁, T₂, ... T_{K});
• eine erste statistische Kennzahl (I1) zu berechnen, die aus dem Mittel, dem Modus und dem Halbwert einer ersten Teilmenge der Vielzahl an spektralen Leistungswerten ausgewählt ist, assoziiert mit einer ersten Teilmenge der Vielzahl an Messzeitsubintervallen;
• eine zweite statistische Kennzahl (I2) zu berechnen, die aus dem Mittel, dem Modus und dem Halbwert einer zweiten Teilmenge der Vielzahl an spektralen Leistungswerten ausgewählt ist, assoziiert mit einer zweiten Teilmenge der Vielzahl an Messzeitsubintervallen, wobei die erste und die zweite Teilmenge der Vielzahl an Messzeitsubintervallen unterschiedlich sind;
• eine Ergebniskennzahl (I_res) zu berechnen, die das Verhältnis zwischen der zweiten statistischen Kennzahl (I2) und der ersten statistischen Kennzahl (I1) ist.

2. Vorrichtung nach Anspruch 1, wobei die Vorrichtung zudem eine elektronische Ausgabevorrichtung (2-11) umfasst, wobei die Verarbeitungseinheit zudem ausgelegt ist, um
• als eine Funktion des Erfassungssignals ein Sauerstoffsättigungssignal zu generieren, das repräsentativ für den Anteil der Sauerstoffsättigung im Blut ist;
• den Wert des Sauerstoffsättigungsanteils mit einem Schwellensauerstoffwert zumindest für die Dauer eines ersten vorgegebenen Zeitintervalls zu vergleichen, das insbesondere zwischen 10 Sekunden und 15 Sekunden enthalten ist;
• den Vergleich für eine Vielzahl an weiteren ersten Zeitintervallen zu wiederholen, bis ein zweites vorgegebenes Zeitintervall erreicht wird, das kleiner oder gleich dem Gesamtmesszeitintervall (T_mt) ist, wobei das zweite Zeitintervall insbesondere gleich 1 Stunde ist, wodurch eine jeweilige Vielzahl an Sauerstoffsättigungsereignissen generiert wird, wobei der Wert des Sauerstoffsättigungsanteils mit dem Schwellensauerstoffwert verglichen wird;
• während des zweiten Zeitintervalls die Zahl der Vielzahl an Sauerstoffsättigungsereignissen zu berechnen, bei denen der Wert des Sauerstoffsättigungsanteils geringer ist als der Schwellensauerstoffwert für mindestens das gesamte jeweilige erste vorgegebene Zeitintervall;
• einen kritischen Wert des Sauerstoffsättigungsanteils zu erfassen, wenn die berechnete Zahl größer oder gleich einem Ereignisschwellenwert ist;
• einen normalen Wert des Sauerstoffsättigungsanteils zu erfassen, wenn die berechnete Zahl kleiner ist als der Ereignisschwellenwert;
• als eine Funktion des Werts der Ergebniskennzahl (I_res) und des erfassten Werts des Sauerstoffsättigungsanteils ein Steuersignal (S_drv) der elektronischen Ausgabevorrichtung (2-11) zu generieren.

3. Vorrichtung (2) nach einem der vorhergehenden Ansprüche, wobei die Verarbeitungseinheit zudem ausgelegt ist, um
• eine Arrhythmie-Kennzahl als eine Funktion der Werte der Vielzahl der gefilterten Proben, repräsentativ für das Interbeat-Intervall, zu berechnen (104-1);
• die Berechnung der Vielzahl an Proben des jeweiligen Leistungsspektrums und der jeweiligen spektralen Leistung für die Vielzahl an weiteren Messsubintervallen zu wiederholen (102, 103, 104, 104-1, 104-2, 105, 106, 107, 108, 109), sodass die Vielzahl an spektralen Leistungswerten und eine entsprechende Vielzahl an Werten der Arrhythmie-Kennzahl (A₁, A₂, ... A_{K}) erhalten werden;
• eine Teilmenge der Vielzahl an Werten der Arrhythmie-Kennzahl auszuwählen (202), aufweisend Werte, die Aufschluss darüber geben, dass keine Arrhythmie vorliegt;
• eine Untervielzahl an spektralen Leistungswerten auszuwählen, assoziiert mit der ausgewählten Teilmenge, definierend einen Anteil, der größer ist als eine Anteilschwelle;
• die erste und die zweite Teilmenge der Vielzahl an spektralen Leistungswerten aus der ausgewählten Untervielzahl an Werten der spektralen Leistung auszuwählen.

4. Vorrichtung (2) nach einem der vorhergehenden Ansprüche, zudem umfassend einen Beschleunigungsmesser mit mindestens einer Achse (2-7), der ausgelegt ist, um ein Beschleunigungssignal (S_AC) zu generieren, das repräsentativ für den Beschleunigungswert der elektronischen Vorrichtung entlang der mindestens einen Achse ist,
wobei die Verarbeitungseinheit (2-2) zudem ausgelegt ist, um das Beschleunigungssignal (S_AC) zu empfangen und darauf basierend eine adaptive Filterung von mindestens einem Teil der Werte der Vielzahl an berechneten Proben durchzuführen, wobei die Verarbeitungseinheit zudem ausgelegt ist, um
• als eine Funktion der Werte des Beschleunigungssignals eine Energiekennzahl zu berechnen, die repräsentativ für die Energie auf der mindestens einen Achse in mindestens einen Zeitintervall ist, das zwischen zwei angrenzenden Herzschlägen enthalten ist;
• als eine Funktion des Vergleichs zwischen dem Wert der berechneten Energiekennzahl und einer Energieschwelle mindestens einen Teil der Werte der Vielzahl der berechneten Proben zu filtern.

5. Vorrichtung (2) nach dem vorhergehenden Anspruch, wobei die Verarbeitungseinheit (2-2) zudem ausgelegt ist, um
- den Wert der Energiekennzahl gegenüber dem Schwellenwert für ein drittes Zeitintervall zu vergleichen;
- als eine Funktion des Werts der Ergebniskennzahl und des Energievergleichs das Steuersignal (S_drv) der elektronischen Ausgabevorrichtung (2-11) zu generieren.

6. Vorrichtung (2) nach dem vorhergehenden Anspruch, wobei die Verarbeitungseinheit zudem ausgelegt ist, um das Steuersignal (S_drv) der elektronischen Ausgabevorrichtung (2-11) als eine Funktion des Werts der Ergebniskennzahl (I_res), des Vergleichs von Energie und des erfassten Werts des Sauerstoffsättigungsanteils zu generieren.

7. Vorrichtung (2) nach Anspruch 5 oder 6, zudem umfassend ein Mikrofon (2-9), das ausgelegt ist, um ein Audiosignal (S_AD) zu generieren, das repräsentativ für Schnarchen ist, wobei die Verarbeitungseinheit (2-2) zudem ausgelegt ist, um
- das Audiosignal zu verarbeiten und daraus das Vorhandensein von Schnarchen zu erfassen;
- als eine Funktion des Werts der Ergebniskennzahl (I_res), des erfassten Werts des Sauerstoffsättigungsanteils und des Werts des Audiosignals (S_AD), das repräsentativ für Schnarchen ist, das Steuersignal (S_drv) der elektronischen Ausgabevorrichtung (2-11) zu generieren.

8. Vorrichtung (2) nach Anspruch 5, 6 oder 7, zudem umfassend ein Mikrofon (2-9), das ausgelegt ist, um das Audiosignal (S_AD) zu generieren, das zudem repräsentativ für eine Emission von Stimmgeräuschen ist, wobei die Verarbeitungseinheit (2-2) zudem ausgelegt ist, um
- das Audiosignal (S_AD) zu verarbeiten und daraus das Vorhandensein von Stimmgeräuschen zu erfassen;
- als eine Funktion des Werts der Ergebniskennzahl (I_res), des Energievergleichs, des erfassten Werts des Sauerstoffsättigungsanteils und des Werts des Audiosignals (S_AD), das repräsentativ für die Emission von Stimmgeräuschen und/oder Schnarchen ist, das Steuersignal (S_drv) der elektronischen Ausgabevorrichtung (2-11) zu generieren.

9. Vorrichtung (2) nach einem der vorhergehenden Ansprüche, wobei
- das Frequenzintervall alternativ zwischen 0,04 Hz und 0,15 Hz oder zwischen 0,15 Hz und 0,4 Hz enthalten ist;
- das Messzeitsubintervall mindestens 5 Minuten beträgt, insbesondere gleich 10 Minuten ist;
- die Summe der Vielzahl an Messzeitsubintervallen mindestens 24 Stunden umfasst, wobei die erste Teilmenge mindestens einen Teil des Tageszeitintervalls umfasst, in dem die zu untersuchende Person wach ist, und die zweite Teilmenge mindestens einen Teil des Nachtzeitintervalls umfasst, in dem die Person schläft, oder umgekehrt.

10. Vorrichtung (2) nach einem der vorhergehenden Ansprüche, wobei sich zwei angrenzende Messzeitsubintervalle mindestens teilweise um einen Anteil überschneiden, der zwischen 10% und 80 % enthalten ist, insbesondere gleich 50 %.

11. Vorrichtung (2) nach einem der vorhergehenden Ansprüche, wobei die Verarbeitungseinheit (2-2) zudem ausgelegt ist, um im Messzeitsubintervall mindestens einen Teil der Werte der Vielzahl an berechneten Proben als eine Funktion des Vergleichs zwischen den Werten der Vielzahl an berechneten Proben und den folgenden Werten zu filtern:
• einem Intervall an Werten, enthalten zwischen einem Interbeat-Mindestwert und einem maximalen Interbeat-Wert, und/oder
• einem maximalen Veränderungswert zwischen zwei angrenzenden Interbeats.

12. Vorrichtung (2) nach einem der vorhergehenden Ansprüche, wobei
- die Vorrichtung (2) ein Gehäuse aufweist, das eine im Wesentlichen kreisförmige Form aufweist, mit einem Durchmesser, der zwischen 20 mm und 30 mm enthalten ist;
- der Sensor (2-1) ein plethysmografischer Sensor ist, insbesondere ein fotoplethysmografischer Sensor vom Reflexionstyp, positioniert in einem Hauptkörper (2a) des Gehäuses und ausgelegt, um ein plethysmografisches Signal zu generieren, das Aufschluss über elektrische Potenziale gibt, die auf dem menschlichen Gewebe erzeugt werden, an dem der Sensor angebracht ist, infolge einer Herzzellenaktivität;
die Vorrichtung (2) zudem einen Sensor (2-10) außerhalb des Hauptkörpers (2a) und ein Verbindungskabel (2b) für den Anschluss des externen Sensors (2-10) am Hauptkörper (2a) umfasst, wobei der externe Sensor (2-10) in einer Ohr-Clip-on-Einheit eingeschlossen ist,
wobei der externe Sensor (2-10) vom Reflexions- oder Penetrationstyp ist und die Verarbeitungseinheit (2-2) ausgelegt ist, um den Anschluss des externen Sensors (2-10) zu erfassen und darauf basierend den Betrieb des internen Sensors (2-1) zu deaktivieren.

13. Elektronisches System (1), umfassend:
- eine elektronische Vorrichtung (2) nach einem der vorhergehenden Ansprüche, wobei die elektronische Vorrichtung zudem ein Sende-Empfangs-Gerät (2-3) für drahtlose Signale umfasst, das ausgelegt ist, um ein drahtloses Signal (S_r_sd) von kurzer Reichweite zu generieren, das Daten mitführt, die von der elektronischen Vorrichtung (2) erfasst oder verarbeitet wurden;
- ein mobiles elektronisches Gerät (3), umfassend einen Bildschirm, das ausgelegt ist, um
• das drahtlose Signal (S_r_sd) von kurzer Reichweite, das die erfassten oder verarbeiteten Daten mitführt, zu empfangen;
• die erfassten Daten zu verarbeiten;
• die verarbeiteten empfangenen Daten und/oder die verarbeiteten erfassten Daten am Bildschirm anzuzeigen.

14. Elektronisches System (1) nach dem vorhergehenden Anspruch, zudem umfassend mindestens einen weiteren Körperdetektor (4, 5, 6), der mit dem mobilen elektronischen Gerät (3) mittels jeweiliger drahtloser Signale mit kurzer Reichweite verbunden ist.

15. Elektronisches System (1) nach dem vorhergehenden Anspruch, umfassend eine Vielzahl an Körperdetektoren (4, 5, 6), ausgewählt aus den folgenden:
- Elektroden für die Elektromyographie;
- Elektroden für die Erfassung eines Elektrokardiogramms;
- Brustgurt und/oder Bauchgurt zum Messen von Atmungsvorgängen;
- nasales Thermoelement zum Messen des Luftstroms beim Einatmen/Ausatmen;
- mit Riemen an den oberen und unteren Gliedmaßen angebrachte Beschleunigungsmesser, um Bewegungen zu erfassen;
- pneumatisch betätigte Vorrichtung zum Messen des arteriellen Blutdrucks, die am Arm oder Handgelenk angebracht werden kann;
- dreikanaliger Mikropolygraf für Elektroenzephalogramm, Elektrookulogramm und Elektromyographie.

16. Verfahren zum Verarbeiten eines Signals (S_V), das repräsentativ für eine Volumenänderung des Blutflusses eines menschlichen Gewebes des lebenden Körpers ist, unter Nutzung einer elektronischen Vorrichtung (2) nach Anspruch 1 bis 12, umfassend die folgenden Schritte:
b) Berechnen (103) einer Vielzahl an Proben (C₁, C₂, ... Cₚ; NN₁, NN₂, ... NNₚ), die repräsentativ für eine jeweilige Vielzahl an Interbeat-Intervallen zwischen angrenzenden Herzschlägen sind, in einem vorgegebenen Messzeitunterintervall (Tᵢ) und als eine Funktion der Werte des Erfassungssignals (S_V);
c) Berechnen (105) eines Leistungsspektrums (Sᵢ) in einem Frequenzintervall als eine Funktion der Werte der Vielzahl an Proben;
d) Berechnen (106) einer spektralen Leistung (Pi) in einem Frequenzsubintervall als eine Funktion des Leistungsspektrums (Si);
e) Wiederholen der Schritte b), c) und d) für eine Vielzahl an weiteren Messzeitsubintervallen (T₁, T₂, ... T_{K}), die einem Gesamtmesszeitintervall (T_mt) angehören, um eine Vielzahl an spektralen Leistungswerten (P₁, P₂, ... P_{K}) zu erhalten, assoziiert mit der Subvielzahl an Messzeitintervallen (T₁, T₂, ... T_{K});
f) Berechnen (111) einer ersten statistischen Kennzahl (I1), ausgewählt aus dem Mittel, dem Modus und dem Halbwert einer ersten Teilmenge der Vielzahl an spektralen Leistungswerten, assoziiert mit einer ersten Teilmenge der Vielzahl an Messzeitsubintervallen;
g) Berechnen (112) einer zweiten statistischen Kennzahl (I2), ausgewählt aus dem Mittel,
dem Modus und dem Halbwert einer zweiten Teilmenge der Vielzahl an spektralen Leistungswerten, assoziiert mit einer zweiten Teilmenge der Vielzahl an Messzeitsubintervallen, wobei die erste und die zweite Teilmenge der Vielzahl an Messzeitsubintervallen unterschiedlich sind;
h) Berechnen (113) einer Ergebniskennzahl (I_res), die das Verhältnis zwischen der zweiten statistischen Kennzahl (I2) und der ersten statistischen Kennzahl (I1) ist.

17. Verarbeitungsverfahren nach Anspruch 16, zudem umfassend vor Schritt h) den Schritt zum Generieren (206) eines Sauerstoffsättigungssignals als eine Funktion des Erfassungssignals, repräsentativ für den Sauerstoffsättigungsanteil im Blut, wobei das Verfahren zudem nach Schritt h) die folgenden Schritte umfasst:
i) Vergleichen des Werts des Sauerstoffsättigungsanteils mit einem Schwellensauerstoffwert zumindest für die Dauer eines ersten vorgegebenen Zeitintervalls, das insbesondere zwischen 10 Sekunden und 15 Sekunden enthalten ist;
j) Wiederholen von Schritt i) für eine Vielzahl an weiteren ersten Zeitintervallen, bis ein zweites vorgegebenes Zeitintervall erreicht wird, das kleiner oder gleich dem Gesamtmesszeitintervall (T_mt) ist, wobei das zweite Zeitintervall insbesondere gleich 1 Stunde ist, wodurch eine jeweilige Vielzahl an Sauerstoffsättigungsereignissen generiert wird, wobei der Wert des Sauerstoffsättigungsanteils mit dem Schwellensauerstoffwert verglichen wird;
k) Berechnen während des zweiten Zeitintervalls der Zahl der Vielzahl an Sauerstoffsättigungsereignissen, bei denen der Wert des Sauerstoffsättigungsanteils geringer ist als der Schwellensauerstoffwert für mindestens das gesamte jeweilige erste vorgegebene Zeitintervall;
I) Erfassen eines kritischen Werts des Sauerstoffsättigungsanteils, wenn die berechnete Zahl größer oder gleich einem Ereignisschwellenwert ist;
m) Erfassen eines normalen Werts des Sauerstoffsättigungsanteils, wenn die berechnete Zahl kleiner ist als der Ereignisschwellenwert;
n) Generieren eines Steuersignals (S_drv) einer elektronischen Ausgabevorrichtung (2-11) als eine Funktion des Werts der Ergebniskennzahl (I_res) und des erfassten Werts des Sauerstoffsättigungsanteils.

18. Verfahren nach Anspruch 16 oder 17, zudem umfassend vor Schritt c) Schritt c1) zum Berechnen (104-1) einer Arrhythmie-Kennzahl als eine Funktion der Werte der Vielzahl der gefilterten Proben, repräsentativ für das Interbeat-Intervall,
wobei das Verfahren das Wiederholen der Schritte b), c) und d) für die Vielzahl der weiteren Messsubintervalle umfasst, sodass die Vielzahl an spektralen Leistungswerten und eine entsprechende Vielzahl an Werten der Arrhythmie-Kennzahl (A₁, A₂, ... A_{K}) erhalten werden,
wobei das Verfahren zudem umfasst:
- Auswählen (202) einer Teilmenge der Vielzahl an Werten der Arrhythmie-Kennzahl, aufweisend Werte, die Aufschluss darüber geben, dass keine Arrhythmie vorliegt;
- Auswählen (203) einer Untervielzahl an spektralen Leistungswerten, assoziiert mit der ausgewählten Teilmenge, definierend einen Anteil, der größer ist als eine Anteilschwelle;
- Auswählen (110-1) der ersten und zweiten Teilmenge der Vielzahl an spektralen Leistungswerten aus der ausgewählten Untervielzahl an Werten der spektralen Leistung.

19. Verfahren nach einem der Ansprüche 16 bis 18, zudem umfassend:
- Empfangen eines Beschleunigungssignals (S_AC), das repräsentativ für den Beschleunigungswert entlang mindestens einer Achse (2-7) ist, und darauf basierend Berechnen einer Energiekennzahl, die repräsentativ für die Energie auf mindestens einer Achse in mindestens einem Zeitintervall ist, enthalten zwischen zwei angrenzenden Herzschlägen;
- Vergleichen des Werts der Energiekennzahl gegenüber dem Schwellenwert für ein drittes Zeitintervall;
- Generieren eines Steuersignals (S_drv) der elektronischen Ausgabevorrichtung (2-11) als eine Funktion des Werts der Ergebniskennzahl und des Energievergleichs.

20. Verfahren nach dem vorhergehenden Anspruch, wobei der Schritt zum Generieren das Generieren des Steuersignals (S_drv) der elektronischen Ausgabevorrichtung (2-11) als eine Funktion des Werts der Ergebniskennzahl (I_res), des Energievergleichs und des erfassten Werts des Sauerstoffsättigungsanteils umfasst.

21. Verfahren nach Anspruch 19 oder 20, zudem umfassend:
- Empfangen eines Audiosignals (S_AD), das repräsentativ für Schnarchen ist;
- Verarbeiten des Audiosignals (S_AD) und daraus Erfassen des Vorhandenseins von Schnarchen;
- Generieren des Steuersignals (S_drv) der elektronischen Ausgabevorrichtung (2-11) als eine Funktion des Werts der Ergebniskennzahl (I_res), des erfassten Werts des Sauerstoffsättigungsanteils und des Werts des Audiosignals (S_AD), das repräsentativ für Schnarchen ist.

22. Verfahren nach Anspruch 19, 20 oder 21, zudem umfassend:
- Empfangen eines Audiosignals (S_AD), das repräsentativ für die Emission von Stimmgeräuschen ist;
- Verarbeiten des Audiosignals (S_AD) und daraus Erfassen des Vorhandenseins von Stimmgeräuschen;
- Generieren des Steuersignals (S_drv) der elektronischen Ausgabevorrichtung (2-11) als eine Funktion des Werts der Ergebniskennzahl (I_res), des Energievergleichs, des erfassten Werts des Sauerstoffsättigungsanteils und des Werts des Audiosignals (S_AD), das repräsentativ für die Emission von Stimmgeräuschen und/oder Schnarchen ist.

23. Verfahren nach einem der Ansprüche 16 bis 22, wobei
- das Frequenzintervall alternativ zwischen 0,04 Hz und 0,15 Hz oder zwischen 0,15 Hz und 0,4 Hz enthalten ist;
- das Messzeitsubintervall mindestens 5 Minuten beträgt, insbesondere gleich 10 Minuten ist;
- der Wert des Gesamtmesszeitintervalls (T_mt) gleich 24 Stunden ist und eine Nachtzeitphase umfasst, in der die untersuchte Person schläft, und eine Tageszeitphase, in der die Person wach ist.

24. Computerprogramm, umfassend Softwarecodeabschnitte, ausgelegt, um die Schritte des Verfahrens nach den Ansprüche 16 bis 23 durchzuführen, wenn das Programm auf mindestens einem Computer ausgeführt wird.

## Revendications

1. Dispositif électronique (2) destiné à la détection automatique de troubles du comportement pendant la phase de sommeil paradoxal et l'apnée du sommeil, le dispositif (2) comprenant :
- un capteur interne (2-1) configuré pour être appliqué en contact avec ou à proximité d'un tissu d'un corps vivant et pour générer un signal d'acquisition (S_V) représentatif d'une variation en volume du flux sanguin d'un tissu humain du corps vivant ;
- un dispositif de sortie électronique (2-11) ;
- une unité de traitement (2-2) **caractérisée en ce que** l'unité de traitement est configurée pour :
• recevoir (102 ; 2-2.2.1) le signal d'acquisition (S_V) et calculer (103 ; 2-2.2.4), dans un sous-intervalle de temps de mesure (Tᵢ) défini et en fonction des valeurs du signal d'acquisition (S_V), une pluralité d'échantillons (C₁, C₂, ... Cₚ; NN₁, NN₂, ... NNₚ) représentative d'une pluralité respective d'intervalles de battement intermédiaire entre des battements de coeur adjacents ;
• calculer (105 ; 2-2.2.5), en fonction des valeurs de la pluralité d'échantillons, un spectre de puissance (Sᵢ) dans un intervalle de fréquence ;
• calculer (106 ; 2-2.2.6), en fonction dudit spectre de puissance (Sᵢ), une puissance spectrale (Pᵢ) dans un sous-intervalle de fréquence ;
• répéter (108, 109, 110) ledit calcul de la pluralité d'échantillons du spectre de puissance respectif et de la puissance spectrale respective pour une pluralité de sous-intervalles de temps de mesure supplémentaires (T₁, T₂, ... T_{K}) appartenant à un intervalle de temps de mesure total (T_mt), de sorte à obtenir une pluralité de valeurs de puissance spectrale (P₁, P₂, ... P_{K}) associée à la pluralité de sous-intervalles de temps de mesure (T₁, T₂, ... T_{K}) ;
• calculer un premier indicateur statistique (I1) sélectionné à partir de la moyenne, du mode et de la médiane d'un premier sous-ensemble de la pluralité de valeurs de puissance spectrale associé à un premier sous-ensemble de la pluralité de sous-intervalles de temps de mesure ;
• calculer un second indicateur statistique (I2) sélectionné à partir de la moyenne, du mode et de la médiane d'un second sous-ensemble de la pluralité de valeurs de puissance spectrale associé à un second sous-ensemble de la pluralité de sous-intervalles de temps de mesure ; dans lequel les premier et second sous-ensembles de la pluralité de sous-intervalles de temps de mesure sont différents ;
• calculer un indice de résultat (I_res) étant le rapport entre le second indicateur statistique (I2) et le premier indicateur statistique (I1).

2. Dispositif selon la revendication 1, le dispositif comprenant de plus un dispositif de sortie électronique (2-11), dans lequel l'unité de traitement est de plus configurée pour :
• générer, en fonction du signal d'acquisition, un signal de saturation en oxygène représentatif du pourcentage de saturation en oxygène dans le sang ;
• comparer la valeur du pourcentage de saturation en oxygène par rapport à une valeur seuil d'oxygène pendant au moins la durée d'un premier intervalle de temps défini, en particulier compris entre 10 et 15 secondes ;
• répéter ladite comparaison pour une pluralité de premiers intervalles de temps supplémentaires jusqu'à atteindre un second intervalle de temps défini étant inférieur ou égal à l'intervalle de temps de mesure total (T_mt), le second intervalle de temps étant égal, en particulier, à 1 heure, générant ainsi une pluralité respective d'événements de saturation en oxygène dans lesquels la valeur du pourcentage de saturation en oxygène est comparée à la valeur seuil d'oxygène ;
• calculer, pendant le second intervalle de temps, le nombre de ladite pluralité d'événements de saturation en oxygène dans lesquels la valeur du pourcentage de saturation en oxygène est inférieure à la valeur seuil d'oxygène pour au moins la totalité du premier intervalle de temps défini respectif ;
• si ledit nombre calculé est supérieur ou égal à une valeur seuil des événements, détecter une valeur critique du pourcentage de saturation en oxygène ;
• si ledit nombre calculé est inférieur à la valeur seuil des événements, détecter une valeur normale du pourcentage de saturation en oxygène ;
• générer, en fonction de la valeur de l'indice de résultat (I_res) et de la valeur détectée du pourcentage de saturation en oxygène, un signal d'actionnement (S_drv) du dispositif de sortie électronique (2-11).

3. Dispositif (2) selon l'une quelconque des revendications précédentes, dans lequel l'unité de traitement est de plus configurée pour :
• calculer (104-1) un indice d'arythmie en fonction des valeurs de la pluralité
d'échantillons filtrés représentatifs de l'intervalle de battement intermédiaire ;
• répéter (102, 103, 104, 104-1, 104-2, 105, 106, 107, 108, 109) le calcul de la pluralité d'échantillons du spectre de puissance respectif et de la puissance spectrale pour la pluralité des sous-intervalles de mesure supplémentaires, de sorte à obtenir la pluralité de valeurs de puissance spectrale et une pluralité correspondante de valeurs de l'indice d'arythmie (A₁, A₂, ... A_{K}) ;
• sélectionner (202) un sous-ensemble de la pluralité de valeurs de l'indice d'arythmie comportant des valeurs indicatives de l'absence d'arythmie ;
• sélectionner une sous-pluralité de valeurs de puissance spectrale associée au dit sous-ensemble sélectionné définissant un pourcentage supérieur à un seuil de pourcentage ;
• sélectionner les premier et second sous-ensembles de la pluralité de valeurs de puissance spectrale à partir de la sous-pluralité sélectionnée des valeurs de la puissance spectrale.

4. Dispositif (2) selon l'une quelconque des revendications précédentes, comprenant de plus un accéléromètre avec au moins un axe (2-7) configuré pour générer un signal d'accélération (S_AC) représentatif de la valeur d'accélération du dispositif électronique le long de l'au moins un axe,
dans lequel l'unité de traitement (2-2) est de plus configurée pour recevoir le signal d'accélération (S_AC) et effectuer, en fonction de celui-ci, un filtrage adaptatif d'au moins une partie des valeurs de la pluralité des échantillons calculés, dans lequel l'unité de traitement est de plus configurée pour :
• calculer, en fonction des valeurs du signal d'accélération, un indice d'énergie représentatif de l'énergie sur l'au moins un axe dans au moins un intervalle de temps compris entre deux battements de coeur adjacents ;
• filtrer, en fonction de la comparaison entre la valeur de l'indice d'énergie calculé et un seuil d'énergie, au moins une partie des valeurs de la pluralité d'échantillons calculés.

5. Dispositif (2) selon la revendication précédente, dans lequel l'unité de traitement (2-2) est de plus configurée pour :
- comparer la valeur de l'indice d'énergie par rapport à la valeur seuil pendant un troisième intervalle de temps ;
• générer, en fonction de la valeur de l'indice de résultat et de ladite comparaison d'énergie, le signal d'actionnement (S_drv) du dispositif de sortie électronique (2-11).

6. Dispositif (2) selon la revendication précédente, dans lequel l'unité de traitement est de plus configurée pour générer, en fonction de la valeur de l'indice de résultat (I_res), de ladite comparaison d'énergie et de la valeur détectée du pourcentage de saturation en oxygène, le signal d'actionnement (S_drv) du dispositif de sortie électronique (2-11).

7. Dispositif (2) selon les revendications 5 ou 6, comprenant de plus un microphone (2-9) configuré pour générer un signal audio (S_AD) représentatif du ronflement, dans lequel l'unité de traitement (2-2) est de plus configurée pour :
- traiter le signal audio et détecter à partir de celui-ci la présence du ronflement ;
- générer, en fonction de la valeur de l'indice de résultat (I_res), de la valeur détectée du pourcentage de saturation en oxygène et de la valeur du signal audio (S_AD) représentatif du ronflement, le signal d'actionnement (S_drv) du dispositif de sortie électronique (2-11).

8. Dispositif (2) selon les revendications 5, 6 ou 7, comprenant de plus un microphone (2-9) configuré pour générer le signal audio (S_AD) représentatif de plus d'une émission de sonorités vocales, dans lequel l'unité de traitement (2-2) est de plus configurée pour :
- traiter le signal audio (S_AD) et détecter à partir de celui-ci la présence de sonorités vocales ;
- générer, en fonction de la valeur de l'indice de résultat (I_res), de ladite comparaison d'énergie, de la valeur détectée du pourcentage de saturation en oxygène et de la valeur du signal audio (S_AD) représentatif de l'émission de sonorités vocales et/ou du ronflement, le signal d'actionnement (S_drv) du dispositif de sortie électronique (2-11).

9. Dispositif (2) selon l'une quelconque des revendications précédentes, dans lequel :
- ledit intervalle de fréquence est compris, alternativement, entre 0,04 et 0,15 Hz ou entre 0,15 et 0,4 Hz ;
- le sous-intervalle de temps de mesure est au moins de 5 minutes, en particulier égal à 10 minutes ;
- la somme de la pluralité de sous-intervalles de temps de mesure comprend au moins 24 heures, dans lequel le premier sous-ensemble comprend au moins une partie de l'intervalle diurne dans lequel le sujet à analyser est réveillé et le second sous-ensemble comprend au moins une partie de l'intervalle nocturne dans lequel le sujet est endormi, ou vice-versa.

10. Dispositif (2) selon l'une quelconque des revendications précédentes, dans lequel deux sous-intervalles de temps de mesure adjacents se recoupent au moins partiellement par un pourcentage compris entre 10 et 80 %, en particulier égal à 50 %.

11. Dispositif (2) selon l'une quelconque des revendications précédentes, dans lequel l'unité de traitement (2-2) est de plus configurée pour filtrer, dans ledit sous-intervalle de temps de mesure, au moins une partie des valeurs de la pluralité d'échantillons calculés, en fonction de la comparaison entre les valeurs de la pluralité d'échantillons calculés et les valeurs suivantes :
• un intervalle de valeurs compris entre une valeur de battement intermédiaire minimum et une valeur de battement intermédiaire maximum ; et/ou
• une valeur maximum de variation entre des battements intermédiaires adjacents.

12. Dispositif (2) selon l'une quelconque des revendications précédentes, dans lequel :
- le dispositif (2) comporte un boîtier ayant une forme substantiellement circulaire et un diamètre compris entre 20 et 30 mm ;
- ledit capteur (2-1) est un capteur pléthysmographique, en particulier un capteur photopléthysmographique de type à réflexion, positionné à l'intérieur d'un corps principal (2a) du boîtier et configuré pour générer un signal pléthysmographique indicatif des potentiels électriques générés sur le tissu humain sur lequel ledit capteur est appliqué du fait de l'activité cellulaire cardiaque ;
le dispositif (2) comprenant de plus un capteur (2-10) externe au corps principal (2a) et un câble de connexion (2b) servant à connecter le capteur externe (2-10) au corps principal (2a), le capteur externe (2-10) étant intégré dans une oreillette à clip,
dans lequel le capteur externe (2-10) est de type à réflexion ou pénétrant, l'unité de traitement (2-2) étant configurée pour détecter la connexion du capteur externe (2-10) et pour désactiver à partir de celui-ci le fonctionnement du capteur interne (2-1).

13. Système électronique (1) comprenant :
- un dispositif électronique (2) selon l'une quelconque des revendications précédentes, le dispositif électronique comprenant de plus un transducteur (2-3) de signaux sans fil configuré pour générer un signal sans fil de courte portée (S_r_sd) transportant des données acquises ou traitées par le dispositif électronique (2) ;
- un dispositif électronique mobile (3) comprenant un écran et configuré pour :
• recevoir ledit signal sans fil de courte portée (S_r_sd) transportant lesdites données acquises ou traitées ;
• traiter les données acquises ;
• afficher à l'écran les données reçues traitées et/ou les données acquises traitées.

14. Système électronique (1) selon la revendication précédente, comprenant de plus au moins un détecteur de corps (4, 5, 6) supplémentaire relié au dispositif électronique mobile (3) au moyen des signaux sans fil de courte portée respectifs.

15. Système électronique (1) selon la revendication précédente, comprenant une pluralité de détecteurs de corps (4, 5, 6) sélectionnés parmi les suivants :
- des électrodes pour l'électromyographie ;
- des électrodes pour l'acquisition d'un électrocardiogramme ;
- un bracelet pour région précordiale et/ou une ceinture abdominale servant à mesurer l'activité respiratoire ;
- un thermocouple nasal destiné à mesurer le débit d'air inhalé / exhalé ;
- des accéléromètres appliqués avec des bandes sur les membres supérieur et inférieur pour détecter les mouvements ;
- un dispositif, actionné de façon pneumatique, destiné à mesurer la pression artérielle et pouvant être appliqué sur le bras ou le poignet ;
- un micro-polygraphe à trois voies pour les électroencéphalogrammes, les électro-oculogrammes et l'électromyographie.

16. Procédé de traitement d'un signal (S_V) représentatif d'une variation en volume du débit sanguin d'un tissu humain du corps vivant en utilisant le dispositif électronique (2) selon les revendications 1 à 12, comprenant les étapes de :
b) calculer (103), dans un sous-intervalle de temps de mesure (Tᵢ) défini et en fonction des valeurs du signal d'acquisition (S_V), une pluralité d'échantillons (C₁, C₂, ... Cₚ; NN₁, NN₂, ... NNₚ) représentative d'une pluralité respective d'intervalles de battement intermédiaire entre des battements de coeur adjacents ;
c) calculer (105), en fonction des valeurs de la pluralité d'échantillons, un spectre de puissance (Sᵢ) dans un intervalle de fréquence ;
d) calculer (106), en fonction dudit spectre de puissance (Si), une puissance spectrale (Pi) dans un sous-intervalle de fréquence ;
e) répéter les étapes b), c) et d) pour une pluralité de sous-intervalles de temps de mesure supplémentaires (T₁, T₂, ... T_{K}) appartenant à un intervalle de temps de mesure total (T_mt), de sorte à obtenir une pluralité de valeurs de puissance spectrale (P₁, P₂, ... P_{K}) associée à la sous-pluralité d'intervalles de temps de mesure (T₁, T₂, ... T_{K}) ;
f) calculer (111) un premier indicateur statistique (I1) sélectionné à partir de la moyenne, du mode et de la médiane d'un premier sous-ensemble de la pluralité de valeurs de puissance spectrale associé à un premier sous-ensemble de la pluralité de sous-intervalles de temps de mesure ;
g) calculer (112) un second indicateur statistique (12) sélectionné à partir de la moyenne, du
mode et de la médiane d'un second sous-ensemble de la pluralité de valeurs de puissance spectrale associé à un second sous-ensemble de la pluralité de sous-intervalles de temps de mesure ; dans lequel les premier et second sous-ensembles de la pluralité de sous-intervalles de temps de mesure sont différents ;
h) calculer (113) un indice de résultat (I_res) étant le rapport entre le second indicateur statistique (I2) et le premier indicateur statistique (I1).

17. Procédé de traitement selon la revendication 16, comprenant de plus, avant l'étape h), l'étape consistant à générer (206), en fonction du signal d'acquisition, un signal de saturation en oxygène représentatif du pourcentage de saturation en oxygène dans le sang, le procédé comprenant de plus, après l'étape h), les étapes de :
i) comparer la valeur du pourcentage de saturation en oxygène par rapport à une valeur seuil d'oxygène pendant au moins la durée d'un premier intervalle de temps défini, en particulier compris entre 10 et 15 secondes ;
j) répéter l'étape i) pour une pluralité de premiers intervalles de temps supplémentaires jusqu'à atteindre un second intervalle de temps défini étant inférieur ou égal à l'intervalle de temps de mesure total (T_mt), en particulier, le second intervalle de temps étant égal à 1 heure, générant ainsi une pluralité respective d'événements de saturation en oxygène dans lesquels la valeur du pourcentage de saturation en oxygène est comparée à la valeur seuil d'oxygène ;
k) calculer, pendant le second intervalle de temps, le nombre de ladite pluralité d'événements de saturation en oxygène dans lesquels la valeur du pourcentage de saturation en oxygène est inférieure à la valeur seuil d'oxygène pendant au moins la totalité du premier intervalle de temps défini respectif ;
l) si ledit nombre calculé est supérieur ou égal à une valeur seuil des événements, détecter une valeur critique du pourcentage de saturation en oxygène ;
m) si ledit nombre calculé est inférieur à la valeur seuil des événements, détecter une valeur normale du pourcentage de saturation en oxygène ;
n) générer, en fonction de la valeur de l'indice de résultat (I_res) et de la valeur détectée du pourcentage de saturation en oxygène, un signal d'actionnement (S_drv) du dispositif de sortie électronique (2-11).

18. Procédé selon les revendications 16 ou 17, comprenant de plus, avant l'étape c), l'étape c1) consistant à calculer (104-1) un indice d'arythmie en fonction des valeurs de la pluralité d'échantillons filtrés représentatifs de l'intervalle de battement intermédiaire,
le procédé comprenant la répétition des étapes b), c) et d) pour la pluralité de sous-intervalles de mesure supplémentaires, de sorte à obtenir la pluralité de valeurs de puissance spectrale et une pluralité correspondante de valeurs de l'indice d'arythmie (A₁, A₂, ... A_{K}),
le procédé comprenant de plus :
- sélectionner (202) un sous-ensemble de la pluralité de valeurs de l'indice d'arythmie comportant des valeurs indicatives de l'absence d'arythmie ;
- sélectionner (203) une sous-pluralité de valeurs de puissance spectrale associée au dit sous-ensemble sélectionné définissant un pourcentage supérieur à un seuil de pourcentage ;
- sélectionner (110-1) les premier et second sous-ensembles de la pluralité de valeurs de puissance spectrale à partir de la sous-pluralité sélectionnée des valeurs de la puissance spectrale.

19. Procédé selon l'une quelconque des revendications de 16 à 18, comprenant de plus :
- recevoir un signal d'accélération (S_AC) représentatif de la valeur d'accélération le long au moins d'un axe (2-7) et calculer à partir de celui-ci un indice d'énergie représentatif de l'énergie sur l'au moins un axe dans au moins un intervalle de temps compris entre deux battements de coeur adjacents ;
- comparer la valeur de l'indice d'énergie par rapport à la valeur seuil pendant un troisième intervalle de temps ;
- générer, en fonction de la valeur de l'indice de résultat et de ladite comparaison d'énergie, le signal d'actionnement (S_drv) du dispositif de sortie électronique (2-11).

20. Procédé selon la revendication précédente, dans lequel l'étape de génération consiste à générer, en fonction de la valeur de l'indice de résultat (I_res), de ladite comparaison d'énergie et de la valeur détectée du pourcentage de saturation en oxygène, le signal d'actionnement (S_drv) du dispositif de sortie électronique (2-11).

21. Procédé selon les revendications 19 ou 20, comprenant de plus :
- recevoir un signal audio (S_AD) représentatif du ronflement ;
- traiter le signal audio (S_AD) et détecter à partir de celui-ci la présence du ronflement ;
- générer, en fonction de la valeur de l'indice de résultat (I_res), de la valeur détectée du pourcentage de saturation en oxygène et de la valeur du signal audio (S_AD) représentatif du ronflement, le signal d'actionnement (S_drv) du dispositif de sortie électronique (2-11).

22. Procédé selon les revendications 19, 20 ou 21, comprenant de plus :
- recevoir un signal audio (S_AD) représentatif de l'émission de sonorités vocales ;
- traiter le signal audio (S_AD) et détecter à partir de celui-ci la présence de sonorités vocales ;
- générer, en fonction de la valeur de l'indice de résultat (I_res), de ladite comparaison d'énergie, de la valeur détectée du pourcentage de saturation en oxygène et de la valeur du signal audio (S_AD) représentatif de l'émission de sonorités vocales et/ou du ronflement, le signal d'actionnement (S_drv) du dispositif de sortie électronique (2-11).

23. Procédé selon les revendications 16 à 22, dans lequel
- ledit intervalle de fréquence est compris, alternativement, entre 0,04 et 0,15 Hz ou entre 0,15 et 0,4 Hz ;
- le sous-intervalle de temps de mesure est au moins de 5 minutes, en particulier égal à 10 minutes ;
- la valeur de l'intervalle de temps de mesure total (T_mt) est égale à 24 heures et comprend une phase nocturne dans laquelle le sujet étant analysé est endormi et une phase diurne dans laquelle le sujet est réveillé.

24. Programme informatique comprenant des parties de code de logiciel adaptées pour effectuer les étapes du procédé selon les revendications 16 à 23, lorsque ledit programme est exécuté sur au moins un ordinateur.
